(19) Europäisches Patentamt
European Patent Office
Office européen des brevets

(11) Veröffentlichungsnummer : **0 465 986 B1**

(12) **EUROPÄISCHE PATENTSCHRIFT**

(45) Veröffentlichungstag der Patentschrift :
**25.05.94 Patentblatt 94/21**

(21) Anmeldenummer : **91110910.6**

(22) Anmeldetag : **02.07.91**

(51) Int. Cl.[5] : **C07C 259/06,** C07C 327/22, A01N 37/30, C07C 259/08, C07C 259/10

# (54) Oxalsäurederivate, ihre Herstellung und sie enthaltende Mittel zur Regulierung des Pflanzenwachstums.

(30) Priorität : **12.07.90 DE 4022265**

(43) Veröffentlichungstag der Anmeldung :
**15.01.92 Patentblatt 92/03**

(45) Bekanntmachung des Hinweises auf die Patenterteilung :
**25.05.94 Patentblatt 94/21**

(84) Benannte Vertragsstaaten :
**BE CH DE DK ES FR GB IT LI NL**

(56) Entgegenhaltungen :
**BULLETIN OF THE CHEMICAL SOCIETY OF JAPAN. Bd. 45, Nr. 12, Dezember 1972,TOKYO JP Seiten 3567 - 71; T.SHINGAKI ET.AL.: 'Photolyses of Ethoxalyl Azide inAlcohols and in Hydrocarbons.Reactions of Ethoxalylnitrene'**

(56) Entgegenhaltungen :
**CHEMICAL ABSTRACTS, vol. 104, no. 9, 3. März 1986, Columbus, Ohio, US, abstract no. 68531V; D.GEFFKEN et al.: "Simple method for preparation of symmetrically substituted N-alkoxylglycoamides", Seite 651, Spalte 2**
**CHEMICAL ABSTRACTS, vol. 114, no. 13, 1. März 1991, Columbus, Ohio, US, abstract no. 116923E; A.AULABAUGH et al.: "Perparation of oxalylhydroxamates as ketol-acid reductoisomerase-inhibiting herbicides andmicrobicides and microbicides", Seite 268, Spalte 2**

(73) Patentinhaber : **BASF Aktiengesellschaft**
**Carl-Bosch-Strasse 38**
**D-67063 Ludwigshafen (DE)**

(72) Erfinder : **Rentzea, Costin, Dr.**
**Richard-Kuhn-Strasse 1-3**
**W-6900 Heidelberg (DE)**
Erfinder : **Rademacher, Wilhelm, Dr.**
**Austrasse 1**
**W-6703 Limburgerhof (DE)**
Erfinder : **Keil, Michael, Dr.**
**Fontanestrasse 4**
**W-6713 Freinsheim (DE)**
Erfinder : **Harreus, Albrecht, Dr.**
**Teichgasse 13**
**W-6700 Ludwigshafen (DE)**

**EP 0 465 986 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Oxalylhydroxamsäurederivate der allgemeinen Formel I,

$R^1$-ON($R^2$)-CO-CO-X-$R^3$ I

in der die Substituenten folgende Bedeutung haben:

X Sauerstoff oder Schwefel;

$R^1$ $C_3$-$C_{18}$-Alkenyl oder $C_3$-$C_8$-Alkinyl, wobei diese Gruppen ein bis fünf Halogenatome tragen können; monocyclisches oder polycyclisches $C_3$-$C_{10}$-Cycloalkyl oder $C_3$-$C_{10}$-Cycloalkylmethyl, wobei diese Ringe ein bis drei $C_1$-$C_4$-Alkylgruppen und/oder einen Phenylring tragen können;
Phenyl, wobei der aromatische Rest ein bis fünf Halogenatome und/oder ein bis drei der folgenden Gruppen tragen kann: Nitro, $C_1$-$C_4$-Aklyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

$R^2$ Wasserstoff, $C_1$-$C_4$-Alkyl, $C_3$-$C_4$-Alkenyl oder Benzyl, wobei der aromatische Rest ein bis fünf Halogenatome und/oder ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

$R^3$ $C_1$-$C_{20}$-Alkyl, welches ein bis fünf Halogenatome und/oder ein bis drei der folgenden Gruppen tragen kann: Cyano, $C_1$-$C_{10}$-Alkoxy, $C_1$-$C_4$-Halogenalkoxy, $C_1$-$C_4$-Alkylthio, $C_1$-$C_4$-Halogenalkylthio, $C_3$-$C_{10}$-Cycloalkyl, $C_3$-$C_{10}$-Cycloalkoxy, $C_3$-$C_{10}$-Cycloalkylthio und $C_3$-$C_{12}$-Alkenyloxy;
$C_3$-$C_{18}$-Alkenyl oder $C_3$-$C_8$-Alkinyl, wobei diese Gruppen ein bis fünf Halogenatome tragen können;
monocyclisches oder polycyclisches $C_3$-$C_{10}$-Cycloalkyl welches ein bis drei $C_1$-$C_4$-Alkylgruppen tragen kann;
Phenyl-$C_1$-$C_4$-alkyl oder Phenoxy-$C_1$-$C_4$-alkyl, wobei die aromatischen Reste ein bis fünf Halogenatome und/oder ein bis drei der folgenden Gruppen tragen können: Nitro, $C_1$-$C_4$-Aklyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

sowie deren landwirtschaftlich brauchbaren Salze.

Außerdem betrifft die Erfindung Verfahren zur Herstellung dieser Verbindungen, sie enthaltende Mittel und Verfahren zur Verwendung von Oxalylhydroxamsäurederivaten der allgemeinen Formel IA,

$R^1$-ON($R^2$)-CO-CO-X-$R^3$ IA

in der die Substituenten folgende Bedeutung haben:

X Sauerstoff oder Schwefel;

$R^1$ $C_1$-$C_{20}$-Alkyl, $C_3$-$C_{18}$-Alkenyl oder $C_3$-$C_8$-Alkinyl, wobei diese Gruppen ein bis fünf Halogenatome tragen können;
$C_2$-$C_8$-Alkyl, welches eine $C_1$-$C_{12}$-Alkoxygruppe trägt;
monocyclisches oder polycyclisches $C_3$-$C_{10}$-Cycloalkyl oder $C_3$-$C_{10}$-Cycloalkylmethyl, wobei diese Ringe ein bis drei $C_1$-$C_4$-Alkylgruppen und/oder einen Phenylring tragen können;
Phenyl oder Phenyl-$C_1$-$C_4$-alkyl, wobei die aromatischen Reste ein bis fünf Halogenatome und/oder ein bis drei der folgenden Gruppen tragen können: Nitro, $C_1$-$C_4$-Aklyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

$R^2$ Wasserstoff, $C_1$-$C_4$-Alkyl, $C_3$-$C_4$-Alkenyl oder Benzyl, wobei der aromatische Rest ein bis fünf Halogenatome und/oder ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

$R^3$ $C_1$-$C_{20}$-Alkyl, welches ein bis fünf Halogenatome und/oder ein bis drei der folgenden Gruppen tragen kann: Cyano, $C_1$-$C_{10}$-Alkoxy, $C_1$-$C_4$-Halogenalkoxy, $C_1$-$C_4$-Alkylthio, $C_1$-$C_4$-Halogenalkylthio, $C_3$-$C_{10}$-Cycloalkyl, $C_3$-$C_{10}$-Cycloalkoxy, $C_3$-$C_{10}$-Cycloalkylthio und $C_3$-$C_{12}$-Alkenyloxy;
$C_3$-$C_{18}$-Alkenyl oder $C_3$-$C_8$-Alkinyl, wobei diese Gruppen ein bis fünf Halogenatome tragen können;
monocyclisches oder polycyclisches $C_3$-$C_{10}$-Cycloalkyl welches ein bis drei $C_1$-$C_4$-Alkylgruppen tragen kann;
Phenyl-$C_1$-$C_4$-alkyl oder Phenoxy-$C_1$-$C_4$-alkyl, wobei die aromatischen Reste ein bis fünf Halogenatome und/oder ein bis drei der folgenden Gruppen tragen können: Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio,

als Pflanzenwachstumsregulatoren.

Oxalylhydroxamsäurederivate sind aus der Literatur als Synthesezwischenprodukte bekannt. Beispielsweise sind (Methoxyamino)oxo-essigsäureethylester, (Ethoxyamino)oxo-essigsäureethylester, (Isopropoxyamino)oxo-essigsäureethylester und (Benzyloxyamino)oxo-essigsäureethylester aus der Fachliteratur bekannt (siehe z.B. M. Takebayashi u.a., Bull. Chem. Soc. Japan, 45, 3567 (1972) und D. Geffken u.a., Chemiker Zeitg., 109, 190 (1985)).

Außerdem wird in der CA-A 2,002,021 die mikrobizide und herbizide Wirkung von Oxalylhydroxamsäurederivaten beschrieben, deren allgemeine Formel die eingangs definierten Verbindungen

I umfaßt. Eine wachstumsregulierende Wirkung derartiger Verbindungen wurde bislang jedoch noch nicht beschrieben.

Aufgabe der vorliegenden Erfindung waren neue wirksame Pflanzenwachstumsregulatoren.

Demgemäß wurden die eingangs definierten Oxalylhydroxamsäurederivate I gefunden. Außerdem wurden Verfahren zur Herstellung dieser Oxalylhydroxam-säurederivate, sie enthaltende Mittel und Verfahren zur Verwendung der ebenfalls eingangs definierten Oxalylhydroxamsäurederivate IA gefunden.

Die Oxalylhydroxamsäurederivate I sind auf verschiedenen Wegen zugänglich. Besonders vorteilhaft erhält man sie nach einem der im nachfolgenden beschriebenen Verfahren A und B.

Verfahren A:

Man erhält die Oxalylhydroxamsäurederivate der Formel I beispielsweise dadurch, daß man ein Oxalylesterhalogenid der allgemeinen Formel II, in an sich bekannter Weise in einem inerten organischen Lösungsmittel in Gegenwart eine Base mit einem Hydroxylamin der allgemeinen Formel III umsetzt.

$$\underset{II}{Hal\text{-}CO\text{-}CO\text{-}X\text{-}R^3} + \underset{III}{R^1\text{-}ON(R^2)\text{-}H} \longrightarrow \underset{I}{R^1\text{-}ON(R^2)\text{-}CO\text{-}CO\text{-}X\text{-}R^3}$$

Hal in der Formel II steht für ein Halogenatom wie Fluor, Chlor, Brom und Jod, vorzugsweise für Chlor oder Brom.

Die Umsetzung erfolgt im allgemeinen bei Temperaturen von -20 bis 120°C, vorzugsweise von 0 bis 80°C.

Als Lösungsmittel eignen sich beispielsweise Halogenkohlenwasserstoffe, insbesondere Chlorkohlenwasserstoffe, z.B. 1,1,2,2-Tetrachlorethylen oder 1,1,2,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, m-, p-Dichlorbenzol, o-, p-, m-Dibrombenzol, o-, m-, p-Chlortoluol, 1,2,4-Trichlorbenzol; Ether, z.B. Ethylpropylether, Methyl-tert.-butylether, n-Butylethylether, Di-n-butylether, Di-isobutylether, Diisoamylether, Diisopropylether, Anisol, Phenethol, Cyclohexylmethylether, Diethylether, Ethylenglykoldimethylether, Tetrahydrofuran, Dioxan, Thioanisol; Nitrokohlenwasserstoffe wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester z.B. Ethylacetat, Acetessigester, Isobutylacetat; Amide, z.B. Formamid, Methylformamid, Dimethylformamid; Ketone, z.B. Aceton, Methylethylketon, gegebenenfalls auch Wasser und entsprechende Gemische in Betracht. Als Lösungsmittel kann auch die Verbindung der Formel III im Überschuß verwendet werden.

Zweckmäßig verwendet man das Lösungsmittel in einer Menge von 100 bis 2 000 Gew.-%, vorzugsweise von 200 bis 700 Gew.-%, bezogen auf Ausgangsstoff II.

Als Basen kommen beispielsweise Kaliumhydroxid, Natriumhydroxid, Kaliumcarbonat, Natriumcarbonat, Lithiumhydroxid, Lithiumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumbicarbonat, Magnesiumacetat, Zinkhydroxid, Zinkoxid, Zinkcarbonat, Zinkbicarbonat, Zinkacetat, Natriumformiat, Natriumacetat, Trimethylamin, Triethylamin, Tripropylamin, Triisopropylamin, Tributylamin, Triisobutylamin, Tri-sec.-butylamin, Tri-tert.-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-4-aminopyridin, N,N-Diethyl-4-aminopyridin, N,N-Dipropyl-4-aminopyridin, N,N-Dipropyl-4-aminopyridin, N-Methylpiperidin, N-Ethylpiperidin, N-Methyl-pyrrolidin, N-Ethylpyrrolidin, N-Methylimidazol, N-Ethylimidazol, N-Methylmorpholin, N-Ethylmorpholin, N-Methylhexamethylenimin, N-Ethylhexamethylenimin, Pyridin, Chinolin, alpha-Picolin, beta-Picolin, Isochinolin, Pyrimidin, Acridin, N,N,N,',N'-Tetramethylethylendiamin, N,N,N'-N'-Tetraethylethlyendiamin, Chinoxalin, N-Propyldiisopropylamin, N,N-Dimethylcyclohexylamin, 2,6-Lutidin, 2,4-Lutidin, Trifurfurylamin, Triethylendiamin in Betracht.

Verfahren B:

Man erhält die Verbindungen der Formel I beispielsweise auch dadurch, daß man ein Oxalylhydroxylamin der allgemeinen Formel IV, in an sich bekannter Weise in einem inerten organischen Lösungsmittel zunächst mit einem Alkylierungsmittel der allgemeinen Formel V umsetzt, und in den Fällen, in denen $R^2$ nicht Wasserstoff bedeutet, das so erhaltene Oxalylhydroxamsäurederivat Ia in an sich bekannter Weise in einem inerten organischen Lösungsmittel mit einem Alkylierungsmittel der allgemeinen Formel VI umsetzt.

$$HON(H)\text{-}CO\text{-}CO\text{-}X\text{-}R^3 \xrightarrow{R^1\text{-}Y} R^1\text{-}ON(H)\text{-}CO\text{-}CO\text{-}X\text{-}R^3 \xrightarrow{R^2\text{-}Y} R^1\text{-}ON(R^2)\text{-}CO\text{-}CO\text{-}X\text{-}R^3$$

IV V Ia VI

Y in den Formeln V und VI steht für eine nucleophil verdrängbare Abgangsgruppe wie Halogen, beispielsweise Chlor, Brom oder Jod oder Alkyl- oder Arylsulfonyl wie p-Toluolsulfonyl.

Die Umsetzung erfolgt im allgemeinen bei Temperaturen von -10 bis 100°C, vorzugsweise von 0 bis 80°C.

Als Lösungsmittel eignen sich beispielsweise die vorstehend bei Verfahren A genannten. Insbesondere kommen die folgenden in Betracht: Toluol, Aceton, Methylethylketon, Dioxan, Tetrahydrofuran, Diethylether, Methylenchlorid, 1,1,1-Trichlorethan und Dimethylformamid.

Als Basen kommen in diesem Verfahren neben den vorstehend genannten Lithiumhydrid und Natriumhydrid in Betracht.

Im Hinblick auf die bestimmungsgemäße Verwendung der Verbindungen IA kommen als Substituenten folgende Reste in Betracht:

X Sauerstoff oder Schwefel, vorzugsweise Sauerstoff;

$R^1$ $C_1$-$C_{20}$-Alkyl, vorzugsweise verzweigtes oder unverzweigtes $C_1$-$C_6$-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethyl-butyl, 2,3-Dimethylbutyl, 3,3-Dimethylbutyl, 1-Ethyl-butyl, 2-Ethylbutyl, 1,1,2-Trimethylpropyl, 1,2,2-Trimethylpropyl, 1-Ethyl-1-methylpropyl und 1-Ethyl-2-methylpropyl, insbesondere verzweigtes oder unverzweigtes $C_1$-$C_4$-Alkyl wie Methyl, Ethyl, Propyl, 1-Methylethyl, Butyl, 1-Methyl-propyl, 2-Methylpropyl und 1,1-Dimethylethyl,

$C_3$-$C_{18}$-Alkenyl, besonders $C_3$-$C_6$-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, vorzugsweise 2-Propenyl, 2-Butenyl und 2-Methyl-2-propenyl, insbesondere 2-Propenyl und 2-Methyl-2-propenyl,

oder

$C_3$-$C_8$-Alkinyl, besonders $C_3$-$C_6$-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl und 2-Butinyl,

wobei diese Gruppen ein bis fünf Halogenatome wie Fluor, Chlor, Brom und Jod, vorzugsweise Fluor, Chlor und Brom, insbesondere Chlor und Brom tragen können;

$C_2$-$C_8$-Alkyl, besonders $C_2$-$C_6$-Alkyl wie vorstehend genannt, welches eine $C_1$-$C_{12}$-Alkoxygruppe, vorzugsweise eine $C_1$-$C_8$-Alkoxygruppe, insbesondere eine $C_1$-$C_6$-Alkoxygruppe wie Methoxy, Ethoxy,

Propyloxy, 1-Methylethoxy, Butoxy, 1-Methylpropyloxy, 2-Methylpropyloxy, 1,1-Dimethylethoxy, Pentyloxy, 1-Methylbutyloxy, 2-Methylbutyloxy, 3-Methylbutyloxy, 1,1-Dimethylpropyloxy, 1,2-Dimethylpropyloxy, 2,2-Dimethylpropyloxy, 1-Ethylpropyloxy, Hexyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethylbutyloxy, 2-Ethylbutyloxy, 1,1,2-Trimethylpropyloxy, 1,2,2-Trimethylpropyloxy, 1-Ethyl-1-methylpropyloxy und 1-Methyl-2-methylpropyloxy trägt;

monocyclisches oder polycyclisches $C_3$-$C_{10}$-Cycloalkyl wie Cyclopropyl, Cyclobutyl, Cyclopentyl, Cyclohexyl, Cycloheptyl, Cyclooctyl, Menthyl, Norbornyl, Adamantyl und Tricyclodecanyl, vorzugsweise Cyclopropyl, Cyclopentyl und Cyclohexyl, insbesondere Cyclohexyl,

oder monocyclisches oder polycyclisches $C_3$-$C_{10}$-Cycloalkylmethyl wie Cyclopropylmethyl, Cyclobutylmethyl, Cyclopentylmethyl, Cyclohexylmethyl, Cycloheptylmethyl und Cyclooctylmethyl, vorzugsweise Cyclopropylmethyl, Cyclopentylmethyl und Cyclohexylmethyl, insbesondere Cyclohexylmethyl,

wobei diese Ringe ein bis drei $C_1$-$C_4$-Alkylgruppen wie Methyl, Ethyl, Propyl, 1-Methylethyl, Butyl, 1-Methylpropyl, 2-Methylpropyl und 1,1-Dimethylethyl, vorzugsweise Methyl, Ethyl und 1-Methylethyl, insbesondere Methyl

und/oder einen Phenylring tragen können;

Phenyl oder Phenyl-$C_1$-$C_4$-alkyl wie Benzyl, 1-Phenylethyl, 2-Phenylethyl, 1-Phenylpropyl, 2-Phenylpropyl, 3-Phenylpropyl, 1-Methyl-1-phenylethyl, 1-Methyl-1-phenylethyl, 1-Phenylbutyl, 2-Phenylbutyl, 3-Phenylbutyl, 4-Phenylbutyl, 1-Methyl-1-phenylpropyl, 1-Methyl-2-phenylpropyl, 1-Methyl-3-phenylpropyl, 2-Methyl-1-phenylpropyl, 2-Methyl-2-phenylpropyl, 2-Methyl-3-phenylpropyl und 1,1-Dimethyl-2-phenylethyl,

wobei

die aromatischen Reste ein bis fünf Halogenatome wie Fluor, Chlor, Brom und Jod, vorzugsweise Fluor, Chlor und Brom und/oder ein bis drei der folgenden Gruppen tragen können: Nitro,

$C_1$-$C_4$-Alkyl wie Methyl, Ethyl, Propyl, 1-Methylethyl, Butyl, 1-Methylpropyl, 2-Methylpropyl und 1,1-Dimethylethyl, vorzugsweise Methyl, Ethyl, 1-Methylethyl und 1,1-Dimethylethyl, insbesondere Methyl und Ethyl,

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl,

$C_1$-$C_4$-Alkoxy wie Methoxy, Ethoxy, Propyloxy, 1-Methylethoxy, Butyloxy, 1-Methylpropyloxy, 2-Methylpropyloxy und 1,1-Dimethylethoxy, vorzugsweise Methoxy und Ethoxy,

$C_1$-$C_4$-Halogenalkoxy, besonders $C_1$-$C_2$-Halogenalkoxy wie Chlormethyloxy, Dichlormethyloxy, Trichlormethyloxy, Fluormethoxy, Difluormethoxy, Trifluormethoxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise Difluormethoxy und Trifluormethoxy,

$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio, vorzugsweise Methylthio, Ethylthio, Propylthio, Butylthio und 2-Methylpropylthio,

und $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio;

$R^2$ Wasserstoff,

$C_1$-$C_4$-Alkyl wie Methyl, Ethyl, Propyl, 1-Methylethyl, Butyl, 1-Methylpropyl, 2-Methylpropyl und 1,1-Dimethylethyl, vorzugsweise Methyl und Ethyl;

$C_3$-$C_4$-Alkenyl wie 2-Propenyl, 2-Butenyl, 3-Butenyl, 1-Methyl-2-propenyl und 2-Methyl-2-propenyl oder Benzyl, wobei der aromatische Rest ein bis fünf Halogenatome wie Fluor, Chlor, Brom und Jod, vorzugsweise Fluor, Chlor und Brom und/oder ein bis drei der folgenden Gruppen tragen kann:

$C_1$-$C_4$-Alkyl wie Methyl, Ethyl, Propyl, 1-Methylethyl, Butyl, 1-Methylpropyl, 2-Methylpropyl und 1,1-Dimethylethyl, vorzugsweise Methyl, Ethyl, 1-Methylethyl und 1,1-Dimethylethyl, insbesondere Methyl und Ethyl,

$C_1$-$C_4$-Halogenalkyl, besonders $C_1$-$C_2$-Halogenalkyl wie Chlormethyl, Dichlormethyl, Trichlormethyl,

Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl,

$C_1$-$C_4$-Alkoxy wie Methoxy, Ethoxy, Propyloxy, 1-Methylethoxy, Butyloxy, 1-Methylpropyloxy, 2-Methylpropyloxy und 1,1-Dimethylethoxy,

$C_1$-$C_4$-Halogenalkoxy, besonders $C_1$-$C_2$-Halogenalkoxy wie Chlormethyloxy, Dichlormethyloxy, Trichlormethyloxy, Fluormethoxy, Difluormethoxy, Trifluormethoxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy,

$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio,

und $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio,

$R^3$ $C_1$-$C_{20}$-Alkyl, bevorzugt verzweigtes oder unverzweigtes $C_1$-$C_{16}$-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethyl-butyl, 2-Ethylbutyl, 1,1,2-Trimethylpropyl, 1,2,2-Trimethylpropyl, 1-Ethyl-1-methylpropyl und 1-Ethyl-2-methylpropyl, besonders verzweigtes oder unverzweigtes C1-C4-Alkyl wie Methyl, Ethyl, Propyl, 1-Methylethyl, Butyl, 1-Methyl-propyl, 2-Methylpropyl und 1,1-Dimethylethyl, Octyl, 2-Ethylhexyl, Nonyl, Decyl, Dodecyl, Tetradecyl und Hexadecyl,

welches ein bis fünf Halogenatome wie Fluor, Chlor, Brom und Jod, vorzugsweise Fluor, Chlor und Brom, insbesondere Fluor und Chlor und/oder ein bis drei der folgenden Gruppen tragen kann:

Cyano,

$C_1$-$C_{10}$-Alkoxy wie Methoxy, Ethoxy, Propyloxy, 1-Methylethoxy, Butyloxy, 1-Methylpropyloxy, 2-Methylpropyloxy, 1,1-Dimethylethoxy, Pentyloxy, 1-Methylbutyloxy, 2-Methylbutyloxy, 3-Methylbutyloxy, 1,1-Dimethylpropyloxy, 1,2-Dimethylpropyloxy, 2,2-Dimethylpropyloxy, 1-Ethylpropyloxy, Hexyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethylbutyloxy, 2-Ethylbutyloxy, 1,1,2-Trimethylpropyloxy, 1,2,2-Trimethylpropyloxy, 1-Ethyl-1-methylpropyloxy, 1-Ethyl-2-methylpropyloxy, Heptyloxy, Octyloxy, 2-Ethylhexyloxy, Nonyloxy und Decyloxy,

$C_1$-$C_4$-Halogenalkoxy, besonders $C_1$-$C_2$-Halogenalkoxy wie Chlormethyloxy, Dichlormethyloxy, Trichlormethyloxy, Fluormethoxy, Difluormethoxy, Trifluormethoxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyl oxy, 2,2,2-Trichlorethyloxy und Pentafluorethyloxy,

$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio, vorzugsweise Methylthio und Ethylthio,

$C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, vorzugsweise Trifluormethylthio,

$C_3$-$C_{10}$-Cycloalkyl, wie Cyclopropyl, Cyclobutyl, Cyclopentyl, Cyclohexyl, Cycloheptyl, Cyclooctyl, Menthyl, Norbornyl, Adamantyl und Tricyclodecanyl, vorzugsweise Cyclopropyl, Cyclopentyl und Cyclohexyl,

$C_3$-$C_{10}$-Cyctoalkoxy wie Cyclopropyloxy, Cyclobutyloxy, Cyclopentyloxy, Cyclohexyloxy, Cycloheptyloxy, Cyclooctyloxy, Menthyloxy, Norbornyloxy, Adamantyloxy und Tricyclodecanyloxy, vorzugsweise Cyclopropyloxy, Cyclopentyloxy und Cyclohexyloxy,

$C_3$-$C_{10}$-Cycloalkylthio wie Cyclopropylthio, Cyclobutylthio, Cyclopentylthio, Cyclohexylthio, Cycloheptylthio, Cyclooctylthio, Menthylthio, Norbornylthio, Adamantylthio und Tricyclodecanylthio, vorzugsweise Cyclohexylthio,

und $C_3$-$C_{12}$-Alkenyloxy, vorzugsweise $C_3$-$C_8$-Alkenyloxy, insbesondere $C_3$-$C_6$-Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,2-Dimethyl-2-butenyloxy, 1,3-Dimethyl-2-butenyloxy, 2,3-Dimethyl-2-butenyloxy, 1,1-Dimethyl-3-butenyloxy, 1,2-Dimethyl-3-butenyloxy, 1,3-Dimethyl-3-butenyloxy, 2,2-Dimethyl-3-butenyloxy, 2,3-Dimethyl-3-butenyloxy, 1-Ethyl-2-butenyloxy, 2-Ethyl-2-butenyloxy, 1-Ethyl-3-butenyloxy, 2-Ethyl-3-butenyloxy, 1,1,2-Trimethyl-2-propenyloxy, 1-Ethyl-1-methyl-2-propenyloxy und 1-Ethyl-2-methyl-2-propenyloxy;

$C_3$-$C_{18}$-Alkenyl, besonders $C_3$-$C_6$-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, vorzugsweise 2-Propenyl, 2-Butenyl und 2-Methyl-2-propenyl,

oder $C_3$-$C_8$-Alkinyl, besonders $C_3$-$C_6$-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Di-methyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl,

wobei diese Gruppen ein bis fünf Halogenatome wie Fluor, Chlor, Brom und Jod, vorzugsweise Fluor, Chlor und Brom, tragen können;

monocyclisches oder polycyclisches $C_3$-$C_{10}$-Cycloalkyl wie Cyclopropyl, Cyclobutyl, Cyclopentyl, Cyclohexyl, Cycloheptyl, Cyclooctyl, Menthyl, Norbornyl, Adamantyl und Tricyclodecanyl, vorzugsweise Cyclopropyl, Cyclopentyl und Cyclohexyl,

welches ein bis drei $C_1$-$C_4$-Alkylgruppen wie Methyl, Ethyl, Propyl, 1-Methylethyl, Butyl, 1-Methylpropyl, 2-Methylpropyl und 1,1-Dimethylethyl, vorzugsweise Methyl, Ethyl undd 1-Methylpropyl, tragen kann;

Phenyl-$C_1$-$C_4$-alkyl wie Benzyl, 1-Phenylethyl, 2-Phenylethyl, 1-Phenylpropyl, 2-Phenylpropyl, 3-Phenylpropyl, 1-Methyl-1-phenylethyl, 1-Methyl-1-phenylethyl, 1-Phenylbutyl, 2-Phenylbutyl, 3-Phenylbutyl, 4-Phenylbutyl, 1-Methyl-1-phenylpropyl, 1-Methyl-2-phenylpropyl, 1-Methyl-3-phenylpropyl, 2-Methyl-1-phenylpropyl, 2-Methyl-2-phenylpropyl, 2-Methyl-3-phenylpropyl und 1,1-Dimethyl-2-phenylethyl, vorzugsweise Benzyl, 2-Phenylethyl und 3-Phenylpropyl,

oder Phenoxy-$C_1$-$C_4$-alkyl, wie Phenoxymethyl, 1-Phenoxyethyl, 2-Phenoxyethyl, 1-Phenoxypropyl, 2-Phenoxypropyl, 3-Phenoxypropyl, 1-Methyl-1-phenoxyethyl, 1-Methyl-1-phenoxyethyl, 1-Phenoxybutyl, 2-Phenoxybutyl, 3-Phenoxybutyl, 4-Phenoxybutyl, 1-Methyl-1-phenoxypropyl, 1-Methyl-2-phenoxypropyl, 1-Methyl-3-phenoxypropyl, 2-Methyl-1-phenoxypropyl, 2-Methyl-2-phenoxypropyl, 2-Methyl-3-phenoxypropyl und 1,1-Dimethyl-2-phenoxyethyl, vorzugsweise 2-Phenoxyethyl,

wobei die aromatischen Reste ein bis fünf Halogenatome wie Fluor, Chlor, Brom und Jod, vorzugsweise Fluor, Chlor und Brom und/oder ein bis drei der folgenden Gruppen tragen können: Nitro,

$C_1$-$C_4$-Aklyl wie Methyl, Ethyl, Propyl, 1-Methylethyl, Butyl, 1-Methylpropyl, 2-Methylpropyl und 1,1-Dimethylethyl, vorzugsweise Methyl,

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluor-

methyl,

$C_1$-$C_4$-Alkoxy wie Methoxy, Ethoxy, Propyloxy, 1-Methylethoxy, Butyloxy, 1-Methylpropyloxy, 2-Methylpropyloxy und 1,1-Dimethylethoxy, vorzugsweise Methoxy und Ethoxy,

$C_1$-$C_4$-Halogenalkoxy, besonders $C_1$-$C_2$-Halogenalkoxy wie Chlormethyloxy, Dichlormethyloxy, Trichlormethyloxy, Fluormethoxy, Difluormethoxy, Trifluormethoxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise Trifluormethoxy und Difluormethoxy,

$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio, vorzugsweise Methylthio

und

$C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, -2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, vorzugsweise 2,2,2-Trichlorethylthio.

Bevorzugt kommen Verbindungen der allgemeinen Formel IA in Betracht, in denen der Rest $R^1$ die folgende Bedeutung hat:

$C_1$-$C_{20}$-Alkyl wie vorstehend im allgemeinen und im besonderen genannt,

$C_3$-$C_{18}$-Alkenyl wie vorstehend im allgemeinen und im besonderen genannt,

oder $C_3$-$C_8$-Alkinyl wie vorstehend im allgemeinen und im besonderen genannt,

wobei diese Gruppen ein bis fünf Halogenatome, wie vorstehend im allgemeinen und im besonderen genannt, tragen können.

Außerdem werden Verbindungen der allgemeinen Formel IA bevorzugt, in denen $R^3$ für einen der folgenden Reste steht:

$C_1$-$C_{20}$-Alkyl wie vorstehend im allgemeinen und im besonderen genannt,

$C_3$-$C_{18}$-Alkenyl wie vorstehend im allgemeinen und im besonderen genannt,

oder $C_3$-$C_8$-Alkinyl wie vorstehend im allgemeinen und im besonderen genannt,

wobei diese Gruppen ein bis fünf Halogenatome, wie vorstehend im allgemeinen und im besonderen genannt, tragen können,

oder

monocyclisches oder polycyclisches $C_3$-$C_{10}$-Cycloalkyl oder $C_3$-$C_{10}$-Cycloalkylmethyl, wie vorstehend im allgemeinen und im besonderen genannt, welches ein bis drei $C_1$-$C_4$-Alkylgruppen, wie vorstehend im allgemeinen und im besonderen genannt, tragen kann.

Desweiteren werden Verbindungen der allgemeinen Formel IA bevorzugt, in denen $R^2$ Wasserstoff oder Methyl bedeutet, und Verbindungen IA in denen X Sauerstoff bedeutet.

Insbesondere werden solche Verbindungen IA bevorzugt, in denen die Substituenten die folgende Bedeutung haben:

X

Sauerstoff;

$R^1$

$C_3$-$C_{18}$-Alkenyl wie vorstehend im allgemeinen und im besonderen genannt, wobei diese Gruppen ein bis fünf Halogenatome, wie vorstehend im allgemeinen und im besonderen genannt, tragen können,

$R^2$

Wasserstoff oder Methyl und

$R^3$

$C_1$-$C_{20}$-Alkyl wie vorstehend im allgemeinen und im besonderen genannt,

$C_3$-$C_{18}$-Alkenyl wie vorstehend im allgemeinen und im besonderen genannt,

oder $C_3$-$C_8$-Alkinyl wie vorstehend im allgemeinen und im besonderen genannt,

wobei diese Gruppen ein bis fünf Halogenatome, wie vorstehend im allgemeinen und im besonderen genannt, tragen können,

oder

momocyclisches oder polycyclisches $C_3$-$C_{10}$-Cycloalkyl oder $C_3$-$C_{10}$-Cycloalkylmethyl, wie vorstehend im allgemeinen und im besonderen genannt, welches ein bis drei $C_1$-$C_4$-Alkylgruppen, wie vorstehend im allgemeinen und im besonderen genannt, tragen kann.

Als Salze der Verbindungen der Formel IA kommen landwirtschaftlich brauchbare Salze, beispielsweise Alkalimetallsalze, insbesondere das Natrium- oder Kaliumsalz, Erdalkalisalze insbesondere das Calcium-, Magnesium- oder Bariumsalz, Mangan-, Kupfer-, Zink- oder Eisensalz sowie Ammonium-, Phosphonium-,

Sulfonium- oder Sulfoxoniumsalze, beispielsweise Ammoniumsalze, Tetraalkylammoniumsalze, Benzyltrialkylammoniumsalze, Trialkylsulfoniumsalze oder Trialkylsulfoniumsalze in Betracht.

Beispiele für bevorzugte Oxalylhydroxamsäurederivate der allgemeinen Formel IA sind in der folgenden Tabelle aufgeführt.

Tabelle

Verbindungen der allgemeinen Formel IA

$$R^1\text{-ON}(R^2)\text{-CO-CO-X-}R^3 \qquad \text{IA}$$

| $R^1$ | $R^2$ | $R^3$ | X |
|---|---|---|---|
| $CH_3$ | $CH_3$ | $C_2H_5$ | O |
| $C_2H_5$ | $C_2H_5$ | $C_2H_5$ | O |
| $C_2H_5$ | $CH_3$ | $C_2H_5$ | O |
| $C_2H_5$ | $C_2H_5$ | $C_2H_5$ | S |
| $C_2H_5$ | $C_2H_5$ | $n-C_3H_7$ | O |
| $C_2H_5$ | $C_2H_5$ | $CH_3$ | O |
| $n-C_3H_7$ | $n-C_3H_7$ | $C_2H_5$ | O |
| $n-C_3H_7$ | $n-C_3H_7$ | $C_2H_5$ | S |
| $CH_3$ | $CH_3$ | $n-C_3H_7$ | O |
| $CH_3$ | $CH_3$ | $n-C_4H_9$ | O |
| $C_2H_5$ | $C_2H_5$ | $n-C_4H_9$ | O |
| $C_2H_5$ | $C_2H_5$ | $i-C_4H_9$ | O |
| $CH_3$ | $CH_3$ | $n-C_6H_{13}$ | O |
| Allyl | Allyl | $n-C_6H_{13}$ | O |
| $CH_3$ | $CH_3$ | $H(CH_2)_4CH(C_2H_5)CH_2$ | O |
| $C_2H_5$ | $C_2H_5$ | $n-C_8H_{17}$ | O |
| $CH_3$ | $CH_3$ | $n-C_8H_{17}$ | O |
| $n-C_3H_7$ | $n-C_3H_7$ | $n-C_8H_{17}$ | O |
| Allyl | Allyl | $n-C_8H_{17}$ | O |
| $CH_3$ | $CH_3$ | $n-C_9H_{19}$ | O |
| $CH_3$ | $CH_3$ | $n-C_{10}H_{21}$ | O |
| $C_2H_5$ | $C_2H_5$ | $n-C_{12}H_{25}$ | O |
| $C_2H_5$ | $C_2H_5$ | $n-C_{12}H_{25}$ | S |
| $CH_3$ | H | $CH_3$ | O |
| $C_2H_5$ | H | $CH_3$ | O |
| $n-C_3H_7$ | H | $CH_3$ | O |
| $i-C_3H_7$ | H | $CH_3$ | O |
| $CH_2=CHCH_2$ | H | $CH_3$ | O |
| $n-C_4H_9$ | H | $CH_3$ | O |
| sek.-$C_4H_9$ | H | $CH_3$ | O |
| tert.-$C_4H_9$ | H | $CH_3$ | O |
| $CH_2CH=CHCH_3$ | H | $CH_3$ | O |
| $CH_2CH=CHCH_3$ | H | $CH_3$ | S |
| $CH_2C(CH_3)=CH_2$ | H | $CH_3$ | O |
| $H_2C=C(Cl)CH_2$ | H | $CH_3$ | O |

Tabelle (Fortsetzung)

| $R^1$ | $R^2$ | $R^3$ | X |
|---|---|---|---|
| $ClCH=CHCH_2$ | H | $CH_3$ | O |
| $H_2C=C(Br)CH_2$ | H | $CH_3$ | O |
| $(CH_3)_2C=CHCH_2$ | H | $CH_3$ | O |
| $n-C_6H_{13}$ | H | $CH_3$ | O |
| $(CH_3)_3CCH_2CH_2$ | H | $CH_3$ | O |
| $n-C_8H_{17}$ | H | $CH_3$ | O |
| $CH_3CH_2CH_2CH_2CH(C_2H_5)CH_2$ | H | $CH_3$ | O |
| $n-C_{14}H_{29}$ | H | $CH_3$ | O |
| Cyclopropylmethyl | H | $CH_3$ | O |
| Cyclopentyl | H | $CH_3$ | O |
| Cyclohexyl | H | $CH_3$ | O |
| 4-Methylcyclohexyl | H | $CH_3$ | O |
| Cyclohexylmethyl | H | $CH_3$ | O |
| 4-tert.-Butylcyclohexyl | H | $CH_3$ | O |
| $n-C_3H_7$ | H | $C_2H_5$ | O |
| $n-C_3H_7$ | H | $C_2H_5$ | S |
| $CH_2=CHCH_2$ | H | $C_2H_5$ | O |
| $CH_3CH=CHCH_2$ | H | $C_2H_5$ | O |
| $ClCH=CHCH_2$ | H | $C_2H_5$ | O |
| $CH_2=C(Cl)CH_2$ | H | $C_2H_5$ | O |
| $CH_2=C(Br)CH_2$ | H | $C_2H_5$ | O |
| $CH_2=C(CH_3)CH_2$ | H | $C_2H_5$ | O |
| $(CH_3)_2C=CHCH_2$ | H | $C_2H_5$ | O |
| $n-C_4H_9$ | H | $C_2H_5$ | O |
| $i-C_4H_9$ | H | $C_2H_5$ | O |
| $Cl-(CH_2)_3$ | H | $C_2H_5$ | O |
| $Cl-(CH_2)_4$ | H | $C_2H_5$ | O |
| $Br-(CH_2)_4$ | H | $C_2H_5$ | O |
| $Cl-(CH_2)_6$ | H | $C_2H_5$ | O |
| $n-C_{10}H_{21}$ | H | $C_2H_5$ | O |
| $n-C_{16}H_{23}$ | H | $C_2H_5$ | O |
| $CH_3$ | H | $n-C_3H_7$ | O |
| $C_2H_5$ | H | $n-C_3H_7$ | O |
| $nC_3H_7$ | H | $n-C_3H_7$ | O |
| $n-C_3H_7$ | H | $n-C_3H_7$ | O |
| $i-C_3H_7$ | H | $n-C_3H_7$ | O |
| $CH_2=CHCH_2$ | H | $n-C_3H_7$ | O |
| $n-C_4H_9$ | H | $n-C_3H_7$ | O |
| $i-C_4H_9$ | H | $n-C_3H_7$ | O |

Tabelle (Fortsetzung)

| $R^1$ | $R^2$ | $R^3$ | X |
|---|---|---|---|
| $CH_3CH{=}CHCH_2$ | H | $n\text{-}C_3H_7$ | O |
| $CH_2{=}C(CH_3)CH_2$ | H | $n\text{-}C_3H_7$ | O |
| $CH_2{=}C(Cl)CH_2$ | H | $n\text{-}C_3H_7$ | O |
| $CH_2{=}C(Br)CH_2$ | H | $n\text{-}C_3H_7$ | O |
| $(CH_3)_2C{=}CHCH_2$ | H | $n\text{-}C_3H_7$ | O |
| $n\text{-}C_{14}H_{29}$ | H | $n\text{-}C_3H_7$ | O |
| $CH_3$ | H | $CH_2CH_2Cl$ | O |
| $C_2H_5$ | H | $CH_2CH_2Cl$ | O |
| $n\text{-}C_3H_7$ | H | $CH_2CH_2Cl$ | O |
| $i\text{-}C_3H_7$ | H | $CH_2CH_2Cl$ | O |
| $CH_2{=}CHCH_2$ | H | $CH_2CH_2Cl$ | O |
| $n\text{-}C_4H_9$ | H | $CH_2CH_2Cl$ | O |
| $i\text{-}C_4H_9$ | H | $CH_2CH_2Cl$ | O |
| $CH_3CH{=}CHCH_2$ | H | $CH_2CH_2Cl$ | O |
| $CH_2{=}C(CH_3)CH_2$ | H | $CH_2CH_2Cl$ | O |
| $CH_2{=}C(Cl)CH_2$ | H | $CH_2CH_2Cl$ | O |
| $CH_2{=}C(Br)CH_2$ | H | $CH_2CH_2Cl$ | O |
| $Cl\text{-}(CH_2)_3$ | H | $CH_2CH_2Cl$ | O |
| $CH_3$ | H | $n\text{-}C_4H_9$ | O |
| $CH_3$ | H | $n\text{-}C_4H_9$ | S |
| $C_2H_5$ | H | $n\text{-}C_4H_9$ | O |
| $n\text{-}C_3H_7$ | H | $n\text{-}C_4H_9$ | O |
| $i\text{-}C_3H_7$ | H | $n\text{-}C_4H_9$ | O |
| $CH_2{=}CHCH_2$ | H | $n\text{-}C_4H_9$ | O |
| $HC{\equiv}CCH_2$ | H | $n\text{-}C_4H_9$ | O |
| $n\text{-}C_4H_9$ | H | $n\text{-}C_4H_9$ | O |
| sek.-$C_4H_9$ | H | $n\text{-}C_4H_9$ | O |
| tert.-$C_4H_9$ | H | $n\text{-}C_4H_9$ | O |
| $CH_3CH{=}CHCH_2$ | H | $n\text{-}C_4H_9$ | O |
| $CH_2{=}C(CH_3)CH_2$ | H | $n\text{-}C_4H_9$ | O |
| $CH_2{=}C(Cl)CH_2$ | H | $n\text{-}C_4H_9$ | O |
| $CH_2{=}C(Br)CH_2$ | H | $n\text{-}C_4H_9$ | O |
| $(CH_3)_2C{=}CHCH_2$ | H | $n\text{-}C_4H_9$ | O |
| Cycloppropyl | H | $n\text{-}C_4H_9$ | O |
| Cyclopropylmethyl | H | $n\text{-}C_4H_9$ | O |
| Cyclopentyl | H | $n\text{-}C_4H_9$ | O |
| Cyclohexyl | H | $n\text{-}C_4H_9$ | O |
| 2-Cyclohexenyl | H | $n\text{-}C_4H_9$ | O |
| Cyclohexylmethyl | H | $n\text{-}C_4H_9$ | O |
| 4-Methylcyclohexyl | H | $n\text{-}C_4H_9$ | O |

Tabelle (Fortsetzung)

| $R^1$ | $R^2$ | $R^3$ | X |
|---|---|---|---|
| 4-tert.-Butylcyclohexyl | H | $n-C_4H_9$ | O |
| 1-Decalyl | H | $n-C_4H_9$ | O |
| 2-Decalyl | H | $n-C_4H_9$ | O |
| 2-Norbornyl | H | $n-C_4H_9$ | O |
| $C_6H_5CH_2$ | H | $n-C_4H_9$ | O |
| $4-F-C_6H_4CH_2$ | H | $n-C_4H_9$ | O |
| $4-Cl-C_6H_4CH_2$ | H | $n-C_4H_9$ | O |
| $2-Cl-C_6H_4CH_2$ | H | $n-C_4H_9$ | O |
| $3-Cl-C_6H_4CH_2$ | H | $n-C_4H_9$ | O |
| $4-Br-C_6H_4CH_2$ | H | $n-C_4H_9$ | O |
| $2,4-Cl_2-C_6H_3CH_2$ | H | $n-C_4H_9$ | O |
| $3,4-Cl_2-C_6H_3CH_2$ | H | $n-C_4H_9$ | O |
| $4-CH_3-C_6H_4CH_2$ | H | $n-C_4H_9$ | O |
| $4-F_3C-C_6H_4CH_2$ | H | $n-C_4H_9$ | O |
| $4-CH_3O-C_6H_4CH_2$ | H | $n-C_4H_9$ | O |
| $4-O_2N-C_6H_4CH_2$ | H | $n-C_4H_9$ | O |
| $4-tert.-C_4H_9-C_6H_4CH_2$ | H | $n-C_4H_9$ | O |
| $CH_3$ | H | $i-C_4H_9$ | O |
| $C_2H_5$ | H | $i-C_4H_9$ | O |
| $n-C_3H_7$ | H | $i-C_4H_9$ | O |
| $i-C_3H_7$ | H | $i-C_4H_9$ | O |
| $CH_2=CHCH_2$ | H | $i-C_4H_9$ | O |
| $HC\equiv CCH_2$ | H | $i-C_4H_9$ | O |
| $H_3CC=CHCH_2$ | H | $i-C_4H_9$ | O |
| $n-C_4H_9$ | H | $i-C_4H_9$ | O |
| $i-C_4H_9$ | H | $i-C_4H_9$ | O |
| $CH_2=C(CH_3)CH_2$ | H | $i-C_4H_9$ | O |
| $CH_2=C(Cl)CH_2$ | H | $i-C_4H_9$ | O |
| $CH_2=C(Br)CH_2$ | H | $i-C_4H_9$ | O |
| $Cl(CH_2)_3$ | H | $i-C_4H_9$ | O |
| $CH_3OCH_2CH_2$ | H | $i-C_4H_9$ | O |
| $CH_3OCH_2CH_2CH_2$ | H | $i-C_4H_9$ | O |
| $C_2H_5OCH_2CH_2$ | H | $i-C_4H_9$ | O |
| $n-C_3H_7OCH_2CH_2$ | H | $i-C_4H_9$ | O |
| $C_2H_5$ | H | $CH_2=CHCH_2$ | O |
| $n-C_3H_7$ | H | $CH_2=CHCH_2$ | O |
| $i-C_3H_7$ | H | $CH_2=CHCH_2$ | O |
| $CH_2=CHCH_2$ | H | $CH_2=CHCH_2$ | O |
| $CH_2=C(CH_3)CH_2$ | H | $CH_2=CHCH_2$ | O |
| $C_2H_5$ | H | $CH_3CH=CHCH_2$ | O |

Tabelle (Fortsetzung)

| $R^1$ | $R^2$ | $R^3$ | X |
|---|---|---|---|
| $n-C_3H_7$ | H | $CH_3CH=CHCH_2$ | O |
| $CH_2=CHCH_2$ | H | $CH_3CH=CHCH_2$ | O |
| $CH_2=C(CH_3)CH_2$ | H | $CH_3CH=CHCH_2$ | O |
| $C_2H_5$ | H | $ClCH=CHCH_2$ | O |
| $n-C_3H_7$ | H | $ClCH=CHCH_2$ | O |
| $CH_2=CHCH_2$ | H | $ClCH=CHCH_2$ | O |
| $CH_2=C(CH_3)CH_2$ | H | $ClCH=CHCH_2$ | O |
| $C_2H_5$ | H | $(CH_3)_2C=CHCH_2$ | O |
| $n-C_3H_7$ | H | $(CH_3)_2C=CHCH_2$ | O |
| $CH_2=CHCH_2$ | H | $(CH_3)_2C=CHCH_2$ | O |
| $C_2H_5$ | H | $C_8H_{17}CH=CHCH_2$ | O |
| $n-C_3H_7$ | H | $C_8H_{17}CH=CHCH_2$ | O |
| $C_2H_5$ | H | $C_{10}H_{21}CH=CHCH_2$ | O |
| $n-C_3H_7$ | H | $C_{10}H_{21}CH=CHCH_2$ | O |
| $CH_3$ | H | $n-C_5H_{11}$ | O |
| $C_2H_5$ | H | $n-C_5H_{11}$ | O |
| $n-C_3H_7$ | H | $n-C_5H_{11}$ | O |
| $i-C_3H_7$ | H | $n-C_5H_{11}$ | O |
| $n-C_4H_9$ | H | $n-C_5H_{11}$ | O |
| $CH_2=CHCH_2$ | H | $n-C_5H_{11}$ | O |
| $CH_2=C(CH_3)CH_2$ | H | $n-C_5H_{11}$ | O |
| $CH_3$ | H | $n-C_6H_{13}$ | O |
| $C_2H_5$ | H | $n-C_6H_{13}$ | O |
| $n-C_3H_7$ | H | $n-C_6H_{13}$ | O |
| $i-C_3H_7$ | H | $n-C_6H_{13}$ | O |
| $CH_2=CHCH_2$ | H | $n-C_6H_{13}$ | O |
| $HC\equiv CCH_2$ | H | $n-C_6H_{13}$ | O |
| $CH_2=C(CH_3)CH_2$ | H | $n-C_6H_{13}$ | O |
| $C_2H_5$ | H | $CH_3(CH_2)_3CH(C_2H_5)CH_2$ | O |
| $n-C_3H_7$ | H | $CH_3(CH_2)_3CH(C_2H_5)CH_2$ | O |
| $i-C_3H_7$ | H | $CH_3(CH_2)_3CH(C_2H_5)CH_2$ | O |
| $CH_2=CHCH_2$ | H | $CH_3(CH_2)_3CH(C_2H_5)CH_2$ | O |
| $CH_2=C(CH_3)CH_2$ | H | $CH_3(CH_2)_3CH(C_2H_5)CH_2$ | O |
| $n-C_4H_9$ | H | $CH_3(CH_2)_3CH(C_2H_5)CH_2$ | O |
| $C_2H_5$ | H | $n-C_8H_{17}$ | O |
| $C_2H_5$ | H | $n-C_9H_{19}$ | O |
| $C_2H_5$ | H | $n-C_{10}H_{21}$ | O |
| $n-C_3H_7$ | H | $n-C_{10}H_{21}$ | O |
| $i-C_3H_7$ | H | $n-C_{10}H_{21}$ | O |
| $CH_2=CHCH_2$ | H | $n-C_{10}H_{21}$ | O |

Tabelle (Fortsetzung)

| $R^1$ | $R^2$ | $R^3$ | X |
|---|---|---|---|
| $CH_2=C(CH_3)CH_2$ | H | $n-C_{10}H_{21}$ | O |
| $C_2H_5$ | H | $n-C_{12}H_{25}$ | O |
| $n-C_3H_7$ | H | $n-C_{12}H_{25}$ | O |
| $i-C_3H_7$ | H | $n-C_{12}H_{25}$ | O |
| $CH_2=CHCH_2$ | H | $n-C_{12}H_{25}$ | O |
| $CH_2=C(CH_3)CH_2$ | H | $n-C_{12}H_{25}$ | O |
| $n-C_4H_9$ | H | $n-C_{12}H_{25}$ | O |
| $C_2H_5$ | H | $n-C_{14}H_{29}$ | O |
| $n-C_3H_7$ | H | $n-C_{14}H_{29}$ | O |
| $i-C_3H_7$ | H | $n-C_{14}H_{29}$ | O |
| $CH_2=CHCH_2$ | H | $n-C_{14}H_{29}$ | O |
| $CH_2=C(CH_3)CH_2$ | H | $n-C_{14}H_{29}$ | O |
| $C_2H_5$ | H | $n-C_{16}H_{33}$ | O |
| $n-C_3H_7$ | H | $n-C_{16}H_{33}$ | O |
| $i-C_3H_7$ | H | $n-C_{16}H_{33}$ | O |
| $CH_2=CHCH_2$ | H | $n-C_{16}H_{33}$ | O |
| $CH_2=C(CH_3)CH_2$ | H | $n-C_{16}H_{33}$ | O |
| $n-C_4H_9$ | H | $n-C_{16}H_{33}$ | O |
| $C_2H_5$ | H | $n-C_{18}H_{37}$ | O |
| $CH_2=CHCH_2$ | H | $n-C_{18}H_{37}$ | O |
| $C_2H_5$ | H | $n-C_{20}H_{41}$ | O |
| $C_2H_5$ | H | $n-C_3H_7O(CH_2)_3$ | O |
| $C_2H_5$ | H | $n-C_3H_7O(CH_2)_4$ | O |
| $C_2H_5$ | H | $n-C_3H_7O(CH_2)_6$ | O |
| $C_2H_5$ | H | $i-C_3H_7O(CH_2)_2$ | O |
| $C_2H_5$ | H | $i-C_3H_7O(CH_2)_4$ | O |
| $C_2H_5$ | H | $n-C_4H_9O(CH_2)_4$ | O |
| $C_2H_5$ | H | $n-C_4H_9O(CH_2)_6$ | O |
| $C_2H_5$ | H | $i-C_4H_9O(CH_2)_6$ | O |
| $C_2H_5$ | H | $i-C_4H_9O(CH_2)_6$ | O |
| $C_2H_5$ | H | sek.-$C_4H_9O(CH_2)_4$ | O |
| $C_2H_5$ | H | $(CH_3)_3CCH_2O(CH_2)_4$ | O |
| $C_2H_5$ | H | $(CH_3)_3CCH_2CH_2O(CH_2)_4$ | O |
| $C_2H_5$ | H | $n-C_6H_{13}O(CH_2)_4$ | O |
| $C_2H_5$ | H | $(CH_3)_2CHCH_2CH(C_2H_5)CH_2O(CH_2)_4$ | O |
| $C_2H_5$ | H | $n-C_8H_{17}O(CH_2)_4$ | O |
| $C_2H_5$ | H | $n-C_9H_{19}O(CH_2)_4$ | O |
| $C_2H_5$ | H | $n-C_{10}H_{21}O(CH_2)_4$ | O |
| $C_2H_5$ | H | cyclo-$C_5H_9O(CH_2)_4$ | O |

Tabelle (Fortsetzung)

| $R^1$ | $R^2$ | $R^3$ | X |
|---|---|---|---|
| $C_2H_5$ | H | cyclo-$C_6H_{11}O(CH_2)_4$ | O |
| $C_2H_5$ | H | $CH_2=CHCH_2O(CH_2)_4$ | O |
| $C_2H_5$ | H | $CH_2=C(CH_3)CH_2O(CH_2)_4$ | O |
| $C_2H_5$ | H | $(CH_3)_2C=CHCH_2O(CH_2)_4$ | O |
| $C_2H_5$ | H | $C_6H_5CH_2$ | O |
| $C_2H_5$ | H | 4-F-$C_6H_4CH_2$ | O |
| $C_2H_5$ | H | 4Cl-$C_6H_4CH_2$ | O |
| $C_2H_5$ | H | $4BrC_6H_4CH_2$ | O |
| $C_2H_5$ | H | $4CH_3C_6H_4CH_2$ | O |
| $C_2H_5$ | H | 4-$CH_3O$-$C_6H_4CH_2$ | O |
| $C_2H_5$ | H | 4-$F_3C$-$C_6H_4CH_2$ | O |
| $C_2H_5$ | H | 4 $O_2N$-$C_6H_4CH_2$ | O |
| $C_2H_5$ | H | 4-tert.-$C_4H_9$-$C_6H_4CH_2$ | O |
| $C_2H_5$ | H | 2,4-$Cl_2$-$C_6H_3CH_2$ | O |
| $C_2H_5$ | H | Cyclopentyl | O |
| $C_2H_5$ | H | Cyclopentylmethyl | O |
| $C_2H_5$ | H | Cyclohexyl | O |
| $C_2H_5$ | H | Cyclohexylmethyl | O |
| $C_2H_5$ | H | 4-Methylcyclohexyl | O |
| $C_2H_5$ | H | 2-Methylcyclohexyl | O |
| $C_2H_5$ | H | 4-Isopropylcyclohexyl | O |
| $C_2H_5$ | H | 4-tert.-Butylcyclohexyl | O |
| $C_2H_5$ | H | 2-Norbornyl | O |
| $C_2H_5$ | H | 1-Decalyl | O |
| $C_2H_5$ | H | 2-Decalyl | O |
| $CH_3$ | H | $C_2H_5$ | O |
| $C_2H_5$ | H | $C_2H_5$ | O |
| i-$C_3H_7$ | H | $C_2H_5$ | O |
| $C_6H_5CH_2$ | H | $C_2H_5$ | O |

Die erfindungsgemäßen herbiziden und wachstumsregulierenden Wirkstoffe IA bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Die Wirkstoffe IA eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber

auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecyl-alkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Wirkstoffe IA können beispielsweise wie folgt formuliert werden:

I. Man vermischt 90 Gewichtsteile der Verbindung Nr. 32 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 17 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 46 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 46 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 17 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 32 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 17 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 46 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

IX. Man vermischt 95 Gewichtsteile der Verbindung Nr. 32 mit 5 Gewichtsteilen N-Methyl-α-pyrrolidon und erhält eine Lösung, die zur Anwendung in Form kleinster Tropfen geeignet ist.

Die Applikation der herbiziden und wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff IA betragen bei herbizider Anwendung je nach Jahreszeit, Bekämpfungsziel, Zielpflanzen und Wachstumsstadium 0,001 bis 5,0, vorzugsweise 0,1 bis 3,0 kg/ha aktive Substanz (a.S.).

Die Wirkstoffe IA können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren z.B. (Samenbeize, Bodenbehandlung, Blattapplikation oder Stamminjektion bei Bäumen)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren der Formel IA im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der Arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Bei Obst- und anderen Bäumen lassen sich mit den Wachstumsregulatoren Schnittkosten einsparen. Außerdem kann die Alternanz von Obstbäumen durch Wachstumsregulatoren gebrochen werden.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die Verbindungen der Formel IA Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird und
- das Mikroklima im Pflanzenbestand durch einen kompakteren Wuchs günstig beeinflußt wird.

Die erfindungsgemäß zu verwendenden Wachstumsregulatoren der Formel IA können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Zur Verbreiterung des Wirkungsspektrums und zur Erzielung synergistischer Effekte können die erfindungsgemäßen Wirkstoffe IA mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3, 1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Sulfonylharnstoffe, Aryloxy-, Heteroaryloxyphenoxypropionsäuren sowie deren Salze, Ester und Amide und andere in Betracht.

Außerdem kann es von Nutzen sein, die Wirkstoffe IA allein oder in Kombination auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Synthesebeispiele

Die in den nachstehenden Synthesebeispielen wiedergegebenen Vorschriften wurden unter entsprechender Abwandlung der Ausgangsverbindungen zur Gewinnung weiterer Wirkstoffe IA benutzt. Die so erhaltenen Verbindungen sind in der nachstehenden Tabelle mit physikalischen Angaben aufgeführt.

Beispiel 1

[(Methoxy)methylamino]oxo-essigsäuremethylester.

1 500 ml Essigsäure wurden bei 25°C nacheinander mit 149,2 g (1,53 Mol) O,N-Dimethylhydroxylaminhydrochlorid, 268,2 g (3,28 Mol) Natriumacetat und 133,2 g (1,09 Mol) Oxalsäuremonomethylester-chlorid versetzt. Nach zwölfstündigem Nachrühren bei 25°C wurde das Gemisch abgesaugt und das Filtrat eingeengt. Der Rückstand wurde mit 500 ml Essigester versetzt, mit 50 ml Wasser gewaschen, über $Na_2SO_4$ getrocknet, im Vakuum eingeengt und anschließend fraktioniert destilliert.

Man erhält 108,8 g (68 % Theorie) [(Methoxy)methylamino]oxo-essigsäuremethylester als farblose Flüssigkeit vom Sdp. 82°C/1 mbar.

Beispiel 2

(Allyloxyamino)oxo-essigsäuremethylester.

13,8 g (0,1 Mol) Kaliumcarbonat und 11,9 g (0,1 Mol) (Hydroxyamino)oxoessigsäuremethylester wurden in 110 ml Dimethylformamid suspendiert, mit 13,3 g (0,11 Mol) Allylbromid versetzt und 10 Stunden bei 60°C nachgerührt. Das Gemisch wurde abgesaugt, im Vakuum eingeengt und der Rückstand in 250 ml Essigester aufgelöst und dreimal mit je 25 ml Wasser gewaschen. Nach Trocknen über $Na_2SO_4$ wurde die Lösung in Essigester im Vakuum eingeengt, zum Schluß bei 80°C und 0,2 mbar.

Man erhält 11,6 g (73 % der Theorie) (Allyloxyamino)oxo-essigsäuremethylester als farbloses Öl, $n_D^{22}$=1,4718.

Tabelle 1

$R^1-ON(R^2)-CO-CO-X-R^3$ IA

| Bsp. Nr. | $R^1$ | $R^2$ | $R^3$ | X | Fp (°C) Sdp. (°C/mbar) Brechungsindex $^1$H-NMR (δ in ppm)* |
|---|---|---|---|---|---|
| 3 | $CH_3$ | $CH_3$ | $C_2H_5$ | O | $n_D^{22}$ 1,4339 |
| 4 | $C_2H_5$ | $C_2H_5$ | $C_2H_5$ | O | $n_D^{22}$ 1,4335 |
| 5 | $C_2H_5$ | $C_2H_5$ | $n-C_3H_7$ | O | $n_D^{23}$ 1,4350 |
| 6 | $C_2H_5$ | $C_2H_5$ | $CH_3$ | O | $n_D^{22}$ 1,4301 |
| 7 | $CH_3$ | $CH_3$ | $n-C_3H_7$ | O | $n_D^{22}$ 1,4358 |
| 8 | $CH_3$ | $CH_3$ | $n-C_4H_9$ | O | $n_D^{22}$ 1,4383 |
| 9 | $CH_3$ | $CH_3$ | $n-C_6H_{13}$ | O | $n_D^{22}$ 1,4422 |
| 10 | $CH_3$ | $CH_3$ | $CH_3CH_2CH_2CH_2CH(C_2H_5)CH_2$ | O | $n_D^{22}$ 1,4466 |
| 11 | $CH_3$ | $CH_3$ | $n-C_8H_{17}$ | O | $n_D^{22}$ 1,4455 |
| 12 | $CH_3$ | $CH_3$ | $n-C_9H_{19}$ | O | $n_D^{22}$ 1,4457 |
| 13 | $CH_3$ | $CH_3$ | $n-C_{10}H_{21}$ | O | $n_D^{23}$ 1,4477 |
| 14 | $CH_3$ | H | $CH_3$ | O | $n_D^{22}$ 1,4547 |
| 15 | $C_2H_5$ | H | $CH_3$ | O | $n_D^{23}$ 1,4549 |
| 16 | $n-C_3H_7$ | H | $CH_3$ | O | $n_D^{23}$ 1,4542 |
| 17 | $i-C_3H_7$ | H | $CH_3$ | O | $n_D^{22}$ 1,4541 |
| 18 | $n-C_4H_9$ | H | $CH_3$ | O | 115-120/0,3 |
| 19 | tert.-$C_4H_9$ | H | $CH_3$ | O | 59- 62 |
| 20 | $CH_2C(CH_3)=CH_2$ | H | $CH_3$ | O | $n_D^{23}$ 1,4776 |
| 21 | $H_2C=C(Cl)CH_2$ | H | $CH_3$ | O | 133-135/0,3 |
| 22 | $ClCH=CHCH_2$ | H | $CH_3$ | O | $n_D^{22}$ 1,4951 |

Tabelle 1 (Fortsetzung)

| Bsp. Nr. | $R^1$ | $R^2$ | $R^3$ | X | Fp (°C) Sdp. (°C/mbar) Brechungsindex $^1$H-NMR (δ in ppm)* |
|---|---|---|---|---|---|
| 23 | $H_2C=C(Br)CH_2$ | H | $CH_3$ | 0 | 135-137/0,3 |
| 24 | $n-C_6H_{13}$ | H | $CH_3$ | 0 | $n_D^{22}$ 1,4581 |
| 25 | $n-C_3H_7$ | H | $C_2H_5$ | 0 | $n_D^{23}$ 1,4532 |
| 26 | $CH_2=C(CH_3)CH_2$ | H | $C_2H_5$ | 0 | $n_D^{23}$ 1,4718 |
| 27 | $CH_3$ | H | $n-C_3H_7$ | 0 | $n_D^{23}$ 1,4538 |
| 28 | $C_2H_5$ | H | $n-C_3H_7$ | 0 | $n_D^{23}$ 1,4538 |
| 29 | $CH_3$ | H | $n-C_4H_9$ | 0 | $n_D^{22}$ 1,4532 |
| 30 | $C_2H_5$ | H | $n-C_4H_9$ | 0 | $n_D^{22}$ 1,4524 |
| 31 | $n-C_3H_7$ | H | $n-C_4H_9$ | 0 | $n_D^{22}$ 1,4529 |
| 32 | $i-C_3H_7$ | H | $n-C_4H_9$ | 0 | $n_D^{22}$ 1,4522 |
| 33 | $CH_2=CHCH_2$ | H | $n-C_4H_9$ | 0 | $n_D^{22}$ 1,4639 |
| 34 | $HC\equiv C-CH_2$ | H | $n-C_4H_9$ | 0 | $n_D^{22}$ 1,4759 |
| 35 | $n-C_4H_9$ | H | $n-C_4H_9$ | 0 | $n_D^{22}$ 1,4548 |
| 36 | tert.-$C_4H_9$ | H | $n-C_4H_9$ | 0 | $n_D^{22}$ 1,4518 |
| 37 | $CH_3-CH=CH-CH_2$ | H | $n-C_4H_9$ | 0 | $n_D^{22}$ 1,4672 |
| 38 | $CH_2=C(CH_3)CH_2$ | H | $n-C_4H_9$ | 0 | $n_D^{22}$ 1,4671 |
| 39 | $CH_2=C(Cl)CH_2$ | H | $n-C_4H_9$ | 0 | $n_D^{22}$ 1,4812 |
| 40 | $CH_2=C(Br)CH_2$ | H | $n-C_4H_9$ | 0 | $n_D^{22}$ 1,4988 |
| 41 | $CH_3$ | H | $n-C_5H_{11}$ | 0 | $n_D^{22}$ 1,4549 |
| 42 | $C_2H_5$ | H | $n-C_5H_{11}$ | 0 | $n_D^{23}$ 1,4537 |
| 43 | $CH_2=C(CH_3)CH_2$ | H | $n-C_5H_{11}$ | 0 | $n_D^{22}$ 1,4555 |
| 44 | $CH_3$ | H | $n-C_6H_{13}$ | 0 | $n_D^{23}$ 1,4551 |

Tabelle 1 (Fortsetzung)

| Bsp. Nr. | $R^1$ | $R^2$ | $R^3$ | X | Fp (°C) Sdp. (°C/mbar) Brechungsindex $^1$H-NMR ($\delta$ in ppm)* |
|---|---|---|---|---|---|
| 45 | $C_2H_5$ | H | n-$C_6H_{13}$ | O | $n_D^{23}$ 1,4539 |
| 46 | $CH_2=C(CH_3)-CH_2$ | H | n-$C_6H_{13}$ | O | $n_D^{23}$ 1,4665 |
| 47 | $C_2H_5$ | H | $CH_3CH_2CH_2CH_2CH(C_2H_5)CH_2$ | O | $n_D^{23}$ 1,4561 |
| 48 | $C_2H_5$ | H | n-$C_{10}H_{21}$ | O | $n_D^{23}$ 1,4565 |
| 49 | $C_2H_5$ | H | n-$C_{12}H_{25}$ | O | 38- 40 |
| 50 | $CH_3$ | H | $C_2H_5$ | O | 90/0,1 |
| 51 | $C_2H_5$ | H | $C_2H_5$ | O | 151/15 |
| 52 | $C_6H_5CH_2$ | H | $C_2H_5$ | O | 85- 87 |
| 53 | $CH_2=C(Br)CH_2$ | H | $C_2H_5$ | O | $n_D^{22}$ 1,5055 |
| 54 | $CH_2=CHCH_2$ | H | $C_2H_5$ | O | $n_D^{22}$ 1,4684 |
| 55 | $(CH_3)_2CHCH_2$ | H | $C_2H_5$ | O | $n_D^{22}$ 1,4515 |
| 56 | $(CH_3)_2CH$ | H | n-$C_3H_7$ | O | $n_D^{22}$ 1,4518 |
| 57 | $CH_2=CHCH_2$ | H | n-$C_3H_7$ | O | $n_D^{22}$ 1,4663 |
| 58 | $(CH_3)_2CHCH_2$ | H | n-$C_3H_7$ | O | $n_D^{22}$ 1,4504 |
| 59 | $C_2H_5$ | H | $(CH_3)_2CHCH_2$ | O | $n_D^{22}$ 1,4501 |
| 60 | $(CH_3)_2CH$ | H | $(CH_3)_2CHCH_2$ | O | $n_D^{22}$ 1,4497 |
| 61 | $CH_2=CHCH_2$ | H | $(CH_3)_2CHCH_2$ | O | $n_D^{22}$ 1,4635 |
| 62 | $ClCH=CHCH_2$ | H | $(CH_3)_2CHCH_2$ | O | 45- 47 |
| 63 | $(CH_3)_2CHCH_2$ | H | $(CH_3)_2CHCH_2$ | O | $n_D^{22}$ 1,4491 |
| 64 | $C_2H_5$ | H | $CH_3(CH_2)_{16}$ | O | $n_D^{22}$ 1,4550 |
| 65 | $(CH_3)_2CH$ | H | $CH_3(CH_2)_{16}$ | O | $n_D^{22}$ 1,4528 |
| 66 | $CH_2=CHCH_2$ | H | $CH_3(CH_2)_{16}$ | O | $n_D^{22}$ 1,4633 |

Tabelle 1 (Fortsetzung)

| Bsp. Nr. | $R^1$ | $R^2$ | $R^3$ | X | Fp (°C) Sdp. (°C/mbar) Brechungsindex $^1$H-NMR (δ in ppm)* |
|---|---|---|---|---|---|
| 67 | $ClCH=CHCH_2$ | H | $CH_3(CH_2)_{16}$ | 0 | 41- 43 |
| 68 | $CH_2=CHCH_2$ | H | $CH_3(CH_2)_3CH(C_2H_5)CH_2$ | 0 | $n_D^{22}$ 1,4651 |
| 69 | $(CH_3)_2CHCH_2$ | H | $CH_3(CH_2)_8$ | 0 | $n_D^{22}$ 1,4543 |
| 70 | $C_2H_5$ | H | $CH_3(CH_2)_8$ | 0 | $n_D^{22}$ 1,4562 |
| 71 | $CH_2=CHCH_2$ | H | $CH_3(CH_2)_8$ | 0 | 35- 37 |
| 72 | $CH_2=CHCH_2$ | H | $CH_3(CH_2)_9$ | 0 | 36- 38 |
| 73 | $CH_2=CHCH_2$ | H | $CH_3(CH_2)_{11}$ | 0 | 46- 47 |
| 74 | $ClCH=CHCH_2$ | H | $CH_3(CH_2)_{11}$ | 0 | 61- 64 |
| 75 | $C_2H_5$ | H | $CH_3(CH_2)_{15}$ | 0 | 61- 62 |
| 76 | $(CH_3)_2CH$ | H | $CH_3(CH_2)_{15}$ | 0 | 68- 70 |
| 77 | $CH_2=CHCH_2$ | H | $CH_3(CH_2)_{15}$ | 0 | 60- 63 |
| 78 | $ClCH=CHCH_2$ | H | $CH_3(CH_2)_{15}$ | 0 | 75- 77 |
| 79 | $(CH_3)_2CH$ | H | $CH_3(CH_2)_3CH(C_2H_5)CH_2$ | 0 | $n_D^{22}$ 1,4558 |
| 80 | $CH_2=C(CH_3)CH_2$ | H | $CH_3(CH_2)_3CH(C_2H_5)CH_2$ | 0 | $n_D^{22}$ 1,4668 |
| 81 | $(CH_3)_2CHCH_2$ | H | $CH_3(CH_2)_3CH(C_2H_5)CH_2$ | 0 | $n_D^{22}$ 1,4555 |
| 82 | $CH_3C(Cl)=CHCH_2$ | H | $n-C_3H_7$ | 0 | 2,16 (s); 4,50 (d); 4,64 (d) |
| 83 | $HC\equiv C-CH_2$ | H | $n-C_3H_7$ | 0 | 2,64 (s); 4,63 (s) |
| 84 | 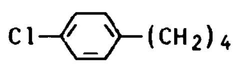 | H | $n-C_3H_7$ | 0 | 3,98 (t); 4,28 (t) |
| 85 | 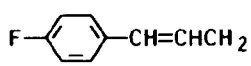 | H | $n-C_3H_7$ | 0 | 6,45 (d); 6,98 (t) |

Tabelle 1 (Fortsetzung)

| Bsp. Nr. | $R^1$ | $R^2$ | $R^3$ | X | Fp (°C) Sdp. (°C/mbar) Brechungsindex $^1$H-NMR (δ in ppm)* |
|---|---|---|---|---|---|
| 86 | 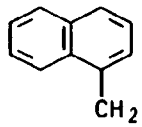 | H | $n-C_3H_7$ | O | 5,64 (s); 8,37 (d) |
| 87 | 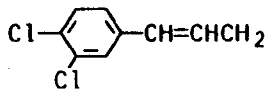 | H | $n-C_3H_7$ | O | 6,42 (d); 7,13 (d) |
| 88 | 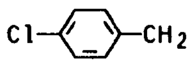 | H | $n-C_3H_7$ | O | 4,97 (s); 7,32 (m) |
| 89 | $(CH_3)_2C{=}CHCH_2C(CH_3){=}CHCH_2$ | H | $n-C_3H_7$ | O | 4,27 (t); 4,49 (d) |
| 90 | 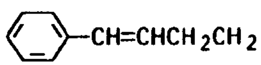 | H | $n-C_3H_7$ | O | 4,03 (t); 6,39 (d) |
| 91 | 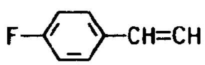 | H | $n-C_3H_7$ | O | 4,62 (d); 6,61 (d) |
| 92 | 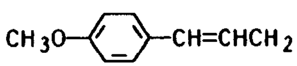 | H | $n-C_3H_7$ | O | 3,81 (s); 6,89 (m) |
| 93 | 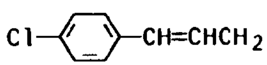 | H | $n-C_3H_7$ | O | 6,45 (d); 7,26 (s) |

Tabelle 1 (Fortsetzung)

| Bsp. Nr. | $R^1$ | $R^2$ | $R^3$ | X | Fp (°C) Sdp. (°C/mbar) Brechungsindex $^1H$-NMR (δ in ppm)* |
|---|---|---|---|---|---|
| 94 | 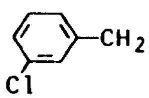 | H | $n-C_3H_7$ | O | 4,29 (t); 4,96 (s) |
| 95 | 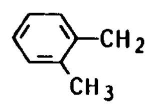 | H | $n-C_3H_7$ | O | 2,45 (s); 5,02 (s) |
| 96 | 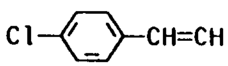 | H | $n-C_3H_7$ | O | 4,63 (d); 6,58 (d) |
| 97 | 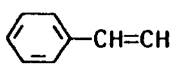 | H | $n-C_3H_7$ | O | 4,61 (d); 6,61 (m) |
| 98 | 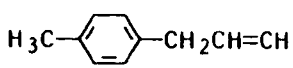 | H | $n-C_3H_7$ | O | 2,31 (s); 4,46 (d) |

Tabelle 1 (Fortsetzung)

| Bsp. Nr. | $R^1$ | $R^2$ | $R^3$ | X | Fp (°C) Sdp. (°C/mbar) Brechungsindex $^1H$-NMR (δ in ppm)* |
|---|---|---|---|---|---|
| 99 | 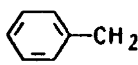$-CH_2CH_2$ | H | $n-C_3H_7$ | O | 3,98 (t); 7,22 (m) |
| 100 | 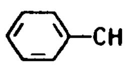$-CH_2$ | H | $n-C_3H_7$ | O | 4,25 (m); 7,24 (m) |
| 101 | 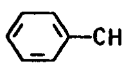$-CH_2CH=CHCH_2$ | H | $n-C_3H_7$ | O | 4,43 (d); 5,70 (m) |

Anwendungsbeispiele

Die wachstumsregulierende Wirkung der Verbindungen der allgemeinen Formel IA ließ sich durch folgende Versuche zeigen:

Die Wirkstoffe wurden

a) als 0,1 %-ige Lösung in Aceton oder

b) als 10 %ige Emulsion in einem Gemisch aus 70 Gew.% Cyclohexanol, 20 Gew.% Nekanil® LN (Lutensol® AP6, Netzmittel mit Emulgier- und Dispergierwirkung auf der Basis ethoxylierter Alkylphenole) und 10 Gew.% Emulphor® EL (Emulan® EL, Emulgator auf der Basis ethoxylierter Fettalkohole)

aufbereitet und entsprechend der gewünschten Konzentration mit Aceton im Fall von a) bzw. mit Wasser im Fall von b) verdünnt.

Als Kulturgefäße dienten Plastikblumentöpfe (Durchmesser ca. 12,5 cm; Volumen ca. 500 ml) mit lehmigem Sand mit etwa 3,0 % Humus als Substrat. Die Samen der Testpflanzen wurden nach Arten getrennt eingesät.

Bei Vorauflaufanwendung wurden die in Wasser suspendierten oder emulgierten Wirkstoffe direkt nach der Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Die Abdeckung bewirkt ein gleichmäßiges Keimen der Testpflanzen.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Die beobachtete wachstumsregulierende Wirkung wurde bei Versuchsende durch die Messung der Wuchshöhe belegt. Die so gewonnenen Meßwerte wurden zur Wuchshöhe von nicht behandelten Pflanzen in Relation gesetzt.

Als Vergleichsubstanz zur Beurteilung der wachstumsregulierenden Wirkung diente N-(2-Clorethyl)-N,N,N-trimethyl-ammoniumchlorid (Beispiel A).

Die in den Gewächshausversuchen verwendeten Pflanzen waren: Sommerweizen (cv. "Ralle"), Sommergerste (cv. "Aramir"), Reis (cv. "Bahia"), Gras (cv. "Apache"), Sonnenblumen (cv. "Spanners Allzweck"), Öllein und Baumwolle (cv. "Stoneville 825").

Bei Nachauflaufanwendung von 0,4 mg/Gefäß zeigten die Verbindungen 2, 17, 18, 20, 21, 23, 26, 30-33, 35, 38-40, 43 und 46 bei Sommerweizen (cv. "Ralle"), die Verbindungen 2, 17, 18, 20-23, 25, 26, 28, 30-35, 37-40, 43-49 und 51 bei Sommergerste (cv. "Aramir") und die Verbindungen 20, 26, 38, 43 und 45 bei Reis (cv. "Bahia") eine bessere Wirkung als der bekannte Wachstumsregulator A.

Bei Nachauflaufanwendung von 1,5 mg/Gefäß zeigten die Verbindungen 20, 26 und 32 bei Baumwolle (cv. "Stoneville 825") und die Verbindung 16 bei Öllein eine bessere Wirkung als der bekannte Wachstumsregulator A.

Bei Nachauflaufanwendung von 6,0 mg/Gefäß zeigten die Verbindungen 82 und 83 bei Sommerweizen (cv. "Ralle") und bei Sommergerste (cv. "Aramir"), die Verbindung 20 bei Gras (cv. "Apache") und die Verbindungen 2, 16-18, 20-23, 25, 26, 28, 30-33, 35, 37-39, 43, 45-49, 51, 58, 82 und 83 bei Sonnenblumen (cv. "Spanners Allzweck") eine bessere Wirkung als der bekannte Wachstumsregulator A.

Gleichlaufend zur Reduzierung des Längenwachstums stieg die Farbintensität der Blätter an. Der erhöhte Chlorophyllgehalt läßt eine erhöhte Photosyntheserate und damit eine Steigerung des Ertrags erwarten.

**Patentansprüche**

1. Oxalylhydroxamsäurederivate der allgemeinen Formel I,

$$R^1\text{-ON}(R^2)\text{-CO-CO-X-}R^3 \qquad \text{I}$$

in der die Substituenten folgende Bedeutung haben:

X Sauerstoff oder Schwefel;

$R^1$ $C_3$-$C_{18}$-Alkenyl oder $C_3$-$C_8$-Alkinyl, wobei diese Gruppen ein bis fünf Halogenatome tragen können;

monocyclisches oder polycyclisches $C_3$-$C_{10}$-Cycloalkyl oder $C_3$-$C_{10}$-Cycloalkylmethyl, wobei diese Ringe ein bis drei $C_1$-$C_4$-Alkylgruppen und/oder einen Phenylring tragen können; oder Phenyl, wobei der aromatische Rest ein bis fünf Halogenatome und/oder ein bis drei der folgenden Gruppen tragen kann: Nitro, $C_1$-$C_4$-Aklyl, $C_1$-$C_4$-Halogenalkyl, $C_1$-$C_4$-Alkoxy, $C_1$-$C_4$-Halo-

genalkoxy, $C_1$-$C_4$-Alkylthio und $C_1$-$C_4$-Halogenalkylthio;

$R^2$ Wasserstoff, $C_1$-$C_4$-Alkyl, $C_3$-$C_4$-Alkenyl oder Benzyl, wobei der aromatische Rest ein bis fünf Halogenatome und/oder ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

$R^3$ $C_1$-$C_{20}$-Alkyl, welches ein bis fünf Halogenatome und/oder ein bis drei der folgenden Gruppen tragen kann: Cyano, $C_1$-$C_{10}$-Alkoxy, $C_1$-$C_4$-Halogenalkoxy, $C_1$-$C_4$-Alkylthio, $C_1$-$C_4$-Halogenalkylthio, $C_3$-$C_{10}$-Cycloalkyl, $C_3$-$C_{10}$-Cycloalkoxy, $C_3$-$C_{10}$-Cycloalkylthio und $C_3$-$C_{12}$-Alkenyloxy;
$C_3$-$C_{18}$-Alkenyl oder $C_3$-$C_8$-Alkinyl, wobei diese Gruppen ein bis fünf Halogenatome tragen können;
monocyclisches oder polycyclisches $C_3$-$C_{10}$-Cycloalkyl welches ein bis drei $C_1$-$C_4$-Alkylgruppen tragen kann;
Phenyl-$C_1$-$C_4$-alkyl oder Phenoxy-$C_1$-$C_4$-alkyl, wobei die aromatischen Reste ein bis fünf Halogenatome und/oder ein bis drei der folgenden Gruppen tragen können: Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

sowie deren landwirtschaftlich brauchbaren Salze.

2. Verbindungen der allgemeinen Formel I gemäß Anspruch 1, in denen $R^1$ $C_2$-$C_{18}$-Alkenyl oder $C_3$-$C_8$-Alkinyl bedeutet, wobei diese Gruppen ein bis fünf Halogenatome tragen können.

3. Verbindungen der allgemeinen Formel I gemäß Anspruch 1, in denen $R^3$ für einen der folgenden Reste steht:
$C_1$-$C_{20}$-Alkyl, $C_3$-$C_{18}$-Alkenyl oder $C_3$-$C_8$-Alkinyl, wobei diese Gruppen ein bis fünf Halogenatome tragen können, oder
momocyclisches oder polycyclisches $C_3$-$C_{10}$-Cycloalkyl oder $C_3$-$C_{10}$-Cycloalkylmethyl, welches ein bis drei $C_1$-$C_4$-Alkylgruppen tragen kann.

4. Verfahren zur Herstellung der Verbindungen der Formel I gemäß Anspruch 1, dadurch gekennzeichnet, daß man ein Oxalylesterhalogenid der allgemeinen Formel II,

$$\text{Hal-CO-CO-X-R}^3 \qquad \text{II}$$

in der Hal für ein Halogenatom steht, in an sich bekannter Weise in einem inerten organischen Lösungsmittel in Gegenwart eine Base mit einem Hydroxylamin der allgemeinen Formel III,

$$\text{R}^1\text{-ON(R}^2\text{)-H} \qquad \text{III}$$

umsetzt.

5. Verfahren zur Herstellung der Verbindungen der Formel I gemäß Anspruch 1, dadurch gekennzeichnet, daß man ein Oxalylhydroxylamin der allgemeinen Formel IV,

$$\text{HON(H)-CO-CO-X-R}^3 \qquad \text{IV}$$

in an sich bekannter Weise in einem inerten organischen Lösungsmittel zunächst mit einem Alkylierungsmittel der allgemeinen Formel V,

$$\text{R}^1\text{-Y} \qquad \text{V}$$

in dem Y für eine nucleophil verdrängbare Abgangsgruppe steht, und in den Fällen, in denen $R^2$ nicht Wasserstoff bedeutet, das so erhaltene Oxalylhydroxamsäurederivat Ia,

$$\text{R}^1\text{-ON(H)-CO-CO-X-R}^3 \qquad \text{Ia}$$

in an sich bekannter Weise in einem inerten organischen Lösungsmittel mit einem Alkylierungsmittel der allgemeinen Formel VI,

$$\text{R}^2\text{-Y} \qquad \text{VI}$$

in dem Y für eine nucleophil verdrängbare Abgangsgruppe steht,
umsetzt.

6. Mittel zur Regulierung des Pflanzenwachstums, enthaltend ein Oxalylhydroxamsäurederivat der allgemeinen Formel I gemäß Anspruch 1 und inerte Zusatzstoffe.

7. Verfahren zu Regulierung des Pflanzenwachstums, dadurch gekennzeichnet, daß man die Pflanzen, ihren Lebensraum und/oder ihre Samen mit einer regulativ wirksamen Menge eines Oxalylhydroxamsäurederivats der allgemeinen Formel IA,

$$\text{R}^1\text{-ON(R}^2\text{)-CO-CO-X-R}^3 \qquad \text{IA}$$

in der die Substituenten folgende Bedeutung haben:

X Sauerstoff oder Schwefel;

$R^1$ $C_1$-$C_{20}$-Alkyl, $C_3$-$C_{18}$-Alkenyl oder $C_3$-$C_8$-Alkinyl, wobei diese Gruppen ein bis fünf Halogenatome tragen können;
$C_2$-$C_8$-Alkyl, welches eine $C_1$-$C_{12}$-Alkoxygruppe trägt;
monocyclisches oder polycyclisches $C_3$-$C_{10}$-Cycloalkyl oder $C_3$-$C_{10}$-Cycloalkylmethyl, wobei diese Ringe ein bis drei $C_1$-$C_4$-Alkylgruppen und/oder einen Phenylring tragen können;
Phenyl oder Phenyl-$C_1$-$C_4$-alkyl, wobei die aromatischen Reste ein bis fünf Halogenatome und/oder ein bis drei der folgenden Gruppen tragen können: Nitro, $C_1$-$C_4$-Aklyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

$R^2$ Wasserstoff, $C_1$-$C_4$-Alkyl, $C_3$-$C_4$-Alkenyl oder Benzyl, wobei der aromatische Rest ein bis fünf Halogenatome und/oder ein bis drei der folgenden Gruppen tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio;

$R^3$ $C_1$-$C_{20}$-Alkyl, welches ein bis fünf Halogenatome und/oder ein bis drei der folgenden Gruppen tragen kann: Cyano, $C_1$-$C_{10}$-Alkoxy, $C_1$-$C_4$-Halogenalkoxy, $C_1$-$C_4$-Alkylthio, $C_1$-$C_4$-Halogenalkylthio, $C_3$-$C_{10}$-Cycloalkyl, $C_3$-$C_{10}$-Cycloalkoxy, $C_3$-$C_{10}$-Cycloalkylthio und $C_3$-$C_{12}$-Alkenyloxy;
$C_3$-$C_{18}$-Alkenyl oder $C_3$-$C_8$-Alkinyl, wobei diese Gruppen ein bis fünf Halogenatome tragen können;
monocyclisches oder polycyclisches $C_3$-$C_{10}$-Cycloalkyl welches ein bis drei $C_1$-$C_4$-Alkylgruppen tragen kann;
Phenyl-$C_1$-$C_4$-alkyl oder Phenoxy-$C_1$-$C_4$-alkyl, wobei die aromatischen Reste ein bis fünf Halogenatome und/oder ein bis drei der folgenden Gruppen tragen können: Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio und $C_1$-$C_4$-Halogenalkylthio,

behandelt.

## Claims

1. An oxalylhydroxamic acid derivative of the formula I

$$R^1\text{-ON}(R^2)\text{-CO-CO-X-}R^3 \qquad I$$

where
X is oxygen or sulfur;
$R^1$ is $C_3$-$C_{18}$-alkenyl or $C_3$-$C_8$-alkynyl, where these groups may carry from one to five halogen atoms; monocyclic or polycyclic $C_3$-$C_{10}$-cycloalkyl or $C_3$-$C_{10}$-cycloalkylmethyl, where these rings may carry from one to three $C_1$-$C_4$-alkyl groups and/or one phenyl ring; or
phenyl, where the aromatic radical may carry from one to five halogen atoms and/or from one to three of the following groups: nitro, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio and $C_1$-$C_4$-haloalkylthio;
$R^2$ is hydrogen, $C_1$-$C_4$-alkyl, $C_3$- or $C_4$-alkenyl or benzyl, where the aromatic radical may carry from one to five halogen atoms and/or from one to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio and $C_1$-$C_4$-haloalkylthio;
$R^3$ is $C_1$-$C_{20}$-alkyl which may carry from one to five halogen atoms and/or from one to three of the following groups: cyano, $C_1$-$C_{10}$-alkoxy, $C_1$-$C_4$-haloalkoxy, $C_1$-$C_4$-alkylthio, $C_1$-$C_4$-haloalkylthio, $C_3$-$C_{10}$-cycloalkyl, $C_3$-$C_{10}$-cycloalkoxy, $C_3$-$C_{10}$-cycloalkylthio and $C_3$-$C_{12}$-alkenyloxy;
$C_3$-$C_{18}$-alkenyl or $C_3$-$C_8$-alkynyl, where these groups may carry from one to five halogen atoms;
monocyclic or polycyclic $C_3$-$C_{10}$-cycloalkyl which may carry from one to three $C_1$-$C_4$-alkyl groups; or
phenyl-$C_1$-$C_4$-alkyl or phenoxy-$C_1$-$C_4$-alkyl, where the aromatic radicals may carry from one to five halogen atoms and/or from one to three of the following groups: nitro, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio and $C_1$-$C_4$-haloalkylthio;
or an agriculturally useful salt thereof.

2. A compound of the formula I as claimed in claim 1, where $R^1$ is $C_3$-$C_{18}$-alkenyl or $C_3$-$C_8$-alkynyl, where these groups may carry from one to five halogen atoms.

3. A compound of the formula I as claimed in claim 1, where $R^3$ is one of the following radicals:
$C_1$-$C_{20}$-alkyl, $C_3$-$C_{18}$-alkenyl or $C_3$-$C_8$-alkynyl, where these groups may carry from one to five halogen atoms, or
monocyclic or polycyclic $C_3$-$C_{10}$-cycloalkyl or $C_3$-$C_{10}$-cycloalkylmethyl which may carry from one to three $C_1$-$C_4$-alkyl groups.

4. A process for the preparation of a compound of the formula I as claimed in claim 1, which comprises reacting an oxalyl ester halide of the formula II

$$Hal\text{-}CO\text{-}CO\text{-}X\text{-}R^3 \qquad II$$

where Hal is halogen, with a hydroxylamine of the formula III

$$R^1\text{-}ON(R^2)\text{-}H \qquad III$$

in a conventional manner in an inert organic solvent in the presence of a base.

5. A process for the preparation of a compound of the formula I as claimed in claim 1, which comprises first reacting an oxalylhydroxylamine of the formula IV

$$HON(H)\text{-}CO\text{-}CO\text{-}X\text{-}R^3 \qquad IV$$

with an alkylating agent of the formula V

$$R^1\text{-}Y \qquad V$$

where Y is a nucleophilically displaceable leaving group, in a conventional manner in an inert organic solvent and, where $R^2$ is not hydrogen, reacting the resulting oxalylhydroxamic acid derivative Ia

$$R^1\text{-}ON(H)\text{-}CO\text{-}CO\text{-}X\text{-}R^3 \qquad Ia$$

with an alkylating agent of the formula VI

$$R^2\text{-}Y \qquad VI$$

where Y is a nucleophilically displaceable leaving group, in a conventional manner in an inert organic solvent.

6. An agent for regulating plant growth, containing an oxalylhydroxamic acid derivative of the formula I as claimed in claim 1 and inert additives.

7. A method of regulating plant growth, wherein the plants, their habitat and/or their seed are (is) treated with a regulative amount of an oxalylhydroxamic acid derivative of the formula IA

$$R^1\text{-}ON(R^2)\text{-}CO\text{-}CO\text{-}X\text{-}R^3 \qquad IA$$

where
X is oxygen or sulfur;
$R^1$ is $C_1$-$C_{20}$-alkyl, $C_3$-$C_{18}$-alkenyl or $C_3$-$C_8$-alkynyl, where these groups may carry from one to five halogen atoms;
$C_2$-$C_8$-alkyl which carried a $C_1$-$C_{12}$-alkoxy group;
monocyclic or polycyclic $C_3$-$C_{10}$-cycloalkyl or $C_3$-$C_{10}$-cycloalkylmethyl, where these rings may carry from one to three $C_1$-$C_4$-alkyl groups and/or one phenyl ring;
phenyl or phenyl-$C_1$-$C_4$-alkyl, where the aromatic radicals may carry from one to five halogen atoms and/or from one to three of the following groups: nitro, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio and $C_1$-$C_4$-haloalkylthio;
$R^2$ is hydrogen, $C_1$-$C_4$-alkyl, $C_3$- or $C_4$-alkenyl or benzyl, where the aromatic radical may carry from one to five halogen atoms and/or from one to three of the following groups: $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio and $C_1$-$C_4$-haloalkylthio;
$R^3$ is $C_1$-$C_{20}$-alkyl which may carry from one to five halogen atoms and/or from one to three of the following groups: cyano, $C_1$-$C_{10}$-alkoxy, $C_1$-$C_4$-haloalkoxy, $C_1$-$C_4$-alkylthio, $C_1$-$C_4$-haloalkylthio, $C_3$-$C_{10}$-cycloalkyl, $C_3$-$C_{10}$-cycloalkoxy, $C_3$-$C_{10}$-cycloalkylthio and $C_3$-$C_{12}$-alkenyloxy;
$C_3$-$C_{18}$-alkenyl or $C_3$-$C_8$-alkynyl, where these groups may carry from one to five halogen atoms;
monocyclic or polycyclic $C_3$-$C_{10}$-cycloalkyl which may carry from one to three $C_1$-$C_4$-alkyl groups;
phenyl-$C_1$-$C_4$-alkyl or phenoxy-$C_1$-$C_4$-alkyl, where the aromatic radicals may carry from one to five halogen atoms and/or from one to three of the following groups: nitro, $C_1$-$C_4$-alkyl, $C_1$-$C_4$-haloalkyl, $C_1$-$C_4$-alkoxy, $C_1$-$C_4$-haloalkoxy, $C_1$-$C_4$-alkylthio and $C_1$-$C_4$-haloalkylthio.

## Revendications

1. Dérivés d'acides oxalylhydroxyamiques de formule générale I

$$R^1\text{-}ON(R^2)\text{-}CO\text{-}CO\text{-}X\text{-}R^3$$

dans laquelle les symboles ont les significations suivantes :
X représente l'oxygène ou le soufre ;
$R^1$ représente un groupe alcényle en $C_3$-$C_{18}$ ou alcynyle en $C_3$-$C_8$, ces groupes pouvant porter 1 à 5 atomes d'halogènes ;
un groupe cycloalkyle en $C_3$-$C_{10}$ ou (cycloalkyle en $C_3$-$C_{10}$)-méthyle mono- ou poly-cyclique, ces cycles

pouvant porter 1 à 3 groupes alkyle en $C_1$-$C_4$ et/ou un noyau phényle ;
ou bien
un groupe phényle dont la partie aromatique peut porter 1 à 5 atomes d'halogène et/ou 1 à 3 des groupes suivants : nitro, alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$ ou halogénoalkylthio en $C_1$-$C_4$ ;
$R^2$ représente l'hydrogène, un groupe alkyle en $C_1$-$C_4$, alcényle en $C_3$-$C_4$ ou benzyle dont la partie aromatique peut porter 1 à 5 atomes d'halogènes et/ou 1 à 3 des groupes suivants : alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$, et halogénoalkylthio en $C_1$-$C_4$ ;
$R^3$ représente un groupe alkyle en $C_1$-$C_{20}$ qui peut porter 1 à 5 atomes d'halogènes et/ou 1 à 3 des groupes suivants : cyano, alcoxy en $C_1$-$C_{10}$, halogénoalcoxy en $C_1$-$C_4$, alkylthio en $C_1$-$C_4$, halogénoalkylthio en $C_1$-$C_4$, cycloalkyle en $C_3$-$C_{10}$, cycloalcoxy en $C_3$-$C_{10}$, cycloalkylthio en $C_3$-$C_{10}$ et alcényloxy en $C_3$-$C_{12}$ ;
un groupe alcényle en $C_3$-$C_{18}$ ou alcynyle en $C_3$-$C_8$, ces groupes pouvant porter 1 à 5 atomes d'halogènes ;
un groupe cycloalkyle en $C_3$-$C_{10}$ mono- ou poly-cyclique qui peut porter 1 à 3 groupes alkyle en $C_1$-$C_4$ ;
un groupe phényl-alkyle en $C_1$-$C_4$ ou phénoxy-alkyle en $C_1$-$C_4$ dont les parties aromatiques peuvent porter 1 à 5 atomes d'halogènes et/ou 1 à 3 des groupes suivants : nitro, alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$ et halogénoalkylthio en $C_1$-$C_4$ ;
ainsi que leurs sels acceptables pour des usages agricoles.

2. Composés de formule générale I de la revendication 1 dans laquelle $R^1$ représente un groupe alcényle en $C_2$-$C_{18}$ ou alcynyle en $C_3$-$C_8$, ces groupes pouvant porter 1 à 5 atomes d'halogènes .

3. Composés de formule générale I de la revendication 1 dans laquelle $R^3$ représente un des groupes suivants alkyle en $C_1$-$C_{20}$, alcényle en $C_3$-$C_{18}$ ou alcynyle en $C_3$-$C_8$, ces groupes pouvant porter 1 à 5 atomes d'halogènes, ou bien
cycloalkyle en $C_3$-$C_{10}$ ou (cycloalkyle en $C_3$-$C_{10}$)-méthyle mono- ou poly-cyclique, ce groupe pouvant porter 1 à 3 groupes alkyle en $C_1$-$C_4$.

4. Procédé de préparation des composés de formule I de la revendication 1, caractérisé en ce que l'on fait réagir un halogénure-ester d'acide oxalique de formule générale II

$$\text{Hal-CO-CO-X-R}^3 \qquad \text{II}$$

dans laquelle Hal représente un atome d'halogène, de manière connue en soi et dans un solvant organique inerte, en présence d'une base, avec une hydroxylamine de formule générale III

$$\text{R}^1\text{-ON(R}^2\text{)-H} \qquad \text{III}$$

5. Procédé de préparation des composés de formule I de la revendication 1, caractérisé en ce que l'on fait réagir une oxalylhydroxylamine de formule générale IV

$$\text{HON(H)-CO-CO-X-R}^3 \qquad \text{IV}$$

de manière connue en soi, dans un solvant organique inerte, d'abord avec un agent alkylant de formule générale V

$$\text{R}^1\text{-Y} \qquad \text{V}$$

dans laquelle Y représente un groupe éliminable nucléophile, puis, dans les cas où $R^2$ ne représente pas l'hydrogène, on fait réagir le dérivé d'acide oxalylhydroxamique ainsi obtenu Ia

$$\text{R}^1\text{-ON(H)-CO-CO-X-R}^3 \qquad \text{Ia}$$

de manière connue en soi, dans un solvant organique inerte, avec un agent alkylant de formule générale VI

$$\text{R}^2\text{-Y} \qquad \text{VI}$$

dans laquelle Y représente un groupe éliminable nucléophile.

6. Produit pour la régulation de la croissance des végétaux contenant un dérivé d'acide oxalylhyroxamique de formule générale I de la revendication 1 et des additifs inertes.

7. Procédé pour la régulation de la croissance des végétaux, caractérisé en ce que l'on traite les végétaux, leur habitat et/ou leurs semences par une quantité régulatrice efficace d'un dérivé d'acide oxalylhydroxamique de formule générale IA

$$\text{R}^1\text{-ON(R}^2\text{)-CO-CO-X-R}^3 \qquad \text{IA}$$

dans laquelle les symboles ont les significations suivantes:
X représente l'oxygène ou le soufre ;
$R^1$ représente un groupe alkyle en $C_1$-$C_{20}$, alcényle en $C_3$-$C_{18}$ ou alcynyle en $C_3$-$C_8$, ces groupes pouvant

porter 1 à 5 atomes d'halogènes ;
un groupe alkyle en $C_2$-$C_8$ qui peut porter un groupe alcoxy en $C_1$-$C_{12}$ ;
un groupe cycloalkyle en $C_3$-$C_{10}$ ou (cycloalkyle en $C_3$-$C_{10}$)-méthyle mono- ou poly-cyclique dont les cycles peuvent porter 1 à 3 groupes alkyle en $C_1$-$C_4$ et/ou un noyau phényle ;
un groupe phényle ou phényl-alkyle en $C_1$-$C_4$ dont les parties aromatiques peuvent porter 1 à 5 atomes d'halogènes et/ou 1 à 3 des groupes suivants : nitro, alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$ et halogénoalkylthio en $C_1$-$C_4$ ;
$R^2$ représente l'hydrogène, un groupe alkyle en $C_1$-$C_4$, alcényle en $C_3$-$C_4$ ou benzyle dont la partie aromatique peut porter 1 à 5 atomes d'halogènes et/ou 1 à 3 des groupes suivants : alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$ et halogénoalkylthio en $C_1$-$C_4$ ;
$R^3$ représente un groupe alkyle en $C_1$-$C_{20}$ qui peut porter 1 à 5 atomes d'halogènes et/ou 1 à 3 groupes suivants cyano, alcoxy en $C_1$-$C_{10}$, halogénoalcoxy en $C_1$-$C_4$, alkylthio en $C_1$-$C_4$, halogénoalkylthio en $C_1$-$C_4$, cycloalkyle en $C_3$-$C_{10}$, cycloalcoxy en $C_3$-$C_{10}$, cycloalkylthio en $C_3$-$C_{10}$ et alcényloxy en $C_3$-$C_{12}$ ;
un groupe alcényle en $C_3$-$C_{18}$ ou alcynyle en $C_3$-$C_8$, ces groupes pouvant porter 1 à 5 atomes d'halogènes ;
un groupe cycloalkyle en $C_3$-$C_{10}$ mono- ou poly-cyclique qui peut porter 1 à 3 groupes alkyle en $C_1$-$C_4$ ;
un groupe phényl-alkyle en $C_1$-$C_4$ ou phénoxy-alkyle en $C_1$-$C_4$ dont les parties aromatiques peuvent porter 1 à 5 atomes d'halogènes et/ou 1 à 3 des groupes suivants : nitro, alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$ et halogénoalkylthio en $C_1$-$C_4$.